(19) **Europäisches Patentamt / European Patent Office / Office européen des brevets**

(11) **EP 4 500 177 B1**

(12) **EUROPEAN PATENT SPECIFICATION**

(45) Date of publication and mention
of the grant of the patent:
**16.07.2025 Bulletin 2025/29**

(21) Application number: **23724672.3**

(22) Date of filing: **28.04.2023**

(51) International Patent Classification (IPC):
**G01N 33/50** (2006.01)     **G01N 33/574** (2006.01)

(52) Cooperative Patent Classification (CPC):
**G01N 33/50; G01N 33/5743;** G01N 2333/9122;
G01N 2800/52

(86) International application number:
**PCT/EP2023/061307**

(87) International publication number:
**WO 2023/209181 (02.11.2023 Gazette 2023/44)**

(54) **THYMIDINE KINASE AS A MARKER FOR IMMUNE CHECKPOINT INHIBITOR EFFICACY**

THYMIDINKINASE ALS MARKER FÜR DIE WIRKSAMKEIT VON
IMMUNCHECKPOINT-INHIBITOREN

LA THYMIDINE KINASE COMME MARQUEUR DE L'EFFICACITÉ DES INHIBITEURS DE
CHECKPOINT IMMUNITAIRE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **29.04.2022 PCT/EP2022/061485**

(43) Date of publication of application:
**05.02.2025 Bulletin 2025/06**

(73) Proprietor: **Biovica International AB
752 37 Uppsala (SE)**

(72) Inventors:
• **BERGQVIST, Mattias
155 30 Nykvarn (SE)**
• **WILLIAMS, Amy Joy
Melrose, Massachusetts 01810 (US)**

(74) Representative: **Potter Clarkson
Chapel Quarter
Mount Street
Nottingham NG1 6HQ (GB)**

(56) References cited:
• LARSSON ANNA-MARIA ET AL: "Serial
evaluation of serum thymidine kinase activity is
prognostic in women with newly diagnosed
metastatic breast cancer", SCIENTIFIC
REPORTS, vol. 10, no. 1, 11 March 2020
(2020-03-11), US, XP093234885, ISSN: 2045-2322,
Retrieved from the Internet <URL:https://www.
nature.com/articles/s41598-020-61416-1> DOI:
10.1038/s41598-020-61416-1
• COSTA SVEDMAN FERNANDA ET AL: "Plasma
Thymidine Kinase Activity as a Novel Biomarker
in Metastatic Melanoma Patients Treated with
Immune Checkpoint Inhibitors", vol. 14, no. 3, 29
January 2022 (2022-01-29), pages 702,
XP093001146, Retrieved from the Internet
<URL:https://www.ncbi.nlm.nih.gov/pmc/
articles/PMC8833501/pdf/cancers-14-00702.pdf>
DOI: 10.3390/cancers14030702
• COSTA SVEDMAN FERNANDA ET AL:
"Supplementary Materials for: Plasma
Thymidine Kinase Activity as a Novel Biomarker
in Metastatic Melanoma Patients Treated with
Immune Checkpoint Inhibitors.", CANCERS, vol.
14, no. 3, 29 January 2022 (2022-01-29), pages 1 -
3, XP093001454, Retrieved from the Internet
<URL:https://www.mdpi.com/article/10.3390/
cancers14030702/s1>

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 500 177 B1

• MCCARTNEY AMELIA ET AL: "Plasma Thymidine Kinase Activity as a Biomarker in Patients with Luminal Metastatic Breast Cancer Treated with Palbociclib within the TREnd Trial", vol. 26, no. 9, 1 May 2020 (2020-05-01), US, pages 2131 - 2139, XP093001168, ISSN: 1078-0432, Retrieved from the Internet <URL:https://aacrjournals.org/clincancerres/article-pdf/26/9/2131/2065988/2131.pdf> DOI: 10.1158/1078-0432.CCR-19-3271

**Description**

The present invention relates to methods and uses associated with determining the prognosis of cancer patients, as well as methods of stratifying those patients for treatment.

Immune checkpoint inhibitors (ICI) are effective in only fractions of cancer patients. Examples of clinically developed ICIs include those targeting programmed cell death-1 (PD-1), programmed cell death ligand-1 (PD-L1), cytotoxic T-lymphocyte antigen-4 (CTLA-4), and lymphocyte activation gene-3 (LAG-3). Antibody drugs targeting these immune checkpoints remove the brakes on T-cell activity, which enables the activation and subsequent proliferation of tumour-reactive T cells which are then able to mount an effective antitumor response. Studies have shown significant increases in the number of T-cells following the first dose of ICI drugs. The level of this increase in T-cells correlates with tumour response and patient outcome [28]. However, measurement of activated and proliferating T-cells requires sorting and isolating the cells from a whole blood population which is complicated, time consuming and utilizes specialized flow cytometry instruments for this purpose. This is not currently used as a standard of care method of predicting patient response to ICIs.

In recent years, effective ICI regimens with CTLA-4 and PD-1 blocking antibodies have emerged for the treatment of multiple cancers, including melanoma [1-6]. These treatments have revolutionised the oncology field, but a considerable fraction of patients do not respond or get lasting effects from these treatments. Significant toxicity can also occur from the treatments, that, in addition, are expensive. It is therefore important to increase knowledge of predictive factors and their efficacy in different patient groups.

Thymidine kinase 1 (TK) is a cytosolic enzyme, a phosphotransferase that plays a pivotal role in DNA synthesis and repair [7]. TK has a key function in DNA synthesis and cell division as it is part of the reaction chain to introduce thymidine into the DNA strand [7]. Dividing cells release TK during mitotic exit, and TK can thus be detected in the blood. Further, elevated TK enzyme activity (TKa) has been measured in blood samples from cancer patients and is associated with tumour proliferation and tumour burden [8]. Circulating levels of TKa, measured with the DiviTum®TKa assay, have been shown to be associated with disease stage, prognosis, and treatment efficacy in several cancer types, including breast, lung, pancreatic, and renal cell cancer [9-15]. Costa Svedman et al. Cancers 14(3) 702 (2022) describes plasma thymidine kinase activity as a biomarker in metastatic melanoma patients treated with immune checkpoint inhibitors. McCartney et al. Clinical Cancer Research 26(9) 2131 (2020) describes plasma thymidine kinase activity as a biomarker in metastatic breast cancer treated with palbociclib within the TREnd trial.

The inventors have surprisingly found that measurement of the activity and/or concentration of thymidine kinase (TK) in patient blood samples, optionally together with blood measurement of the activity and/or concentration of lactate dehydrogenase (LDH), before and during treatment with immune checkpoint inhibitors (ICIs) can be used to provide a prognosis for subsequent treatment with ICIs. Accordingly, this allows it to be determined if the ICI the patient has been given or is under consideration to be given, is not working or will not work effectively. A key benefit of knowing quickly that an ICI drug is ineffective is that these drugs are very expensive and potentially very toxic, so it is in the patient's interest not to begin taking or to stop taking an ineffective drug as soon as possible and to instead potentially be administered an alternative therapy.

A first aspect of the invention provides a method for determining the prognosis of an individual having cancer and currently being treated with one or more immune checkpoint inhibitors, the method comprising or consisting of the steps of:

   a) providing a sample obtained from the individual prior to treatment with one or more immune checkpoint inhibitors;
   b) measuring the activity and/or concentration of thymidine kinase (TK) in the sample;
   c) providing a sample obtained from the individual 1-4 weeks after first treatment with the one or more immune checkpoint inhibitors; and
   d) measuring the activity and/or concentration of thymidine kinase (TK) in the sample provided in step (c)

wherein the activity and/or concentration of thymidine kinase (TK) measured in step (b) and (d) is indicative of the prognosis of the individual if further treated with one or more immune checkpoint inhibitors, wherein the prognosis of the individual if further treated with one or more checkpoint inhibitors is positive when the activity and/or concentration of thymidine kinase (TK) measured in step (d) is increased compared to the activity and/or concentration of thymidine kinase (TK) measured in step (b).

By "thymidine kinase (TK)" we refer to thymidine kinase 1 (TK1). Thymidine Kinase 1 is a key cell cycle-regulated enzyme important for nucleotide metabolism during DNA synthesis. TK1 catalyzes the conversion of thymidine to deoxythymidine monophosphate, which is further phosphorylated to di- and triphosphates preceding its incorporation into DNA. The activity of TK1 is low or absent in resting cells, however increases significantly as cells divide and replicate. Thus, its presence in cells is an indicator of active cell proliferation. TK1 diffuses from proliferating cells to the bloodstream and its activity can be measured in serum or plasma samples from blood. DiviTum®TKa is a highly sensitive and precise blood-based assay for measuring TK activity (TKa) in serum or plasma samples.

Immune checkpoint inhibitors (ICIs) are a class of drugs that block proteins called checkpoints that are made by certain types of immune system cells (such as T cells) and some cancer cells. These checkpoints function generally as negative regulators of the immune system. Upon activation of the checkpoint, the immune cells are inhibited from eliciting a cytotoxic response. These checkpoint proteins are crucial in maintaining the balance between autoimmunity and self-tolerance. However, tumours can often upregulate expression of immune checkpoints, creating an immunosuppressive environment and allowing tumour cells to escape from immune system-mediated destruction. A range of immune checkpoint inhibitors have been developed to treat cancer. The most clinically developed of these are programmed cell death-1 (PD-1), programmed cell death ligand-1 (PD-L1), cytotoxic T-lymphocyte antigen-4 (CTLA-4), lymphocyte activation gene-3 (LAG-3), and T-cell immunoglobulin mucin-3 (TIM-3) inhibitor therapies. Antibody drugs targeting these immune checkpoints remove the brakes on T-cell activity, which enables the activation and subsequent proliferation of tumour-reactive T cells which are then able to mount an effective antitumor response. Studies have shown significant increases in the number of T-cells following the first dose of ICI drugs. The level of this increase in T-cells correlates with tumour response and patient outcome (Kim et al. Clin Cancer Res; 25(7) 2020).

Particular patients may be considered for treatment with immune checkpoint inhibitors when they express immune checkpoint inhibitor receptors (checkpoint proteins) on their cancer cells, or express those receptors at higher levels than healthy individuals, which may be determined by routine methods such as diagnostic assays measuring the level of immune checkpoint inhibitor receptor expression. An example of such a cancer is melanoma, as is the subject of the Example described herein, however the methods of the invention are applicable to other cancers which would normally be considered for treatment with one or more checkpoint inhibitors.

By "prognosis of the individual if subsequently treated with one or more immune checkpoint inhibitors", we include the expected effectiveness of the treatment with one or more immune checkpoint inhibitors, including the progression-free survival (PFS) and overall survival (OS) time of the individual if they are subsequently treated with one or more immune checkpoint inhibitors.

By treatment with one or more check point inhibitors, we include treatment with a single ICI (ICI monotherapy) or treatment with different types of ICI (ICI combination therapy).

The ICI treatment and/or subsequent ICI treatment may be delivered alone or together with an additional non-ICI cancer therapy. In one particular embodiment the ICI treatment and/or subsequent ICI treatment is treatment with one or more ICI alone and without additional non-ICI cancer therapy.

Samples are obtained from the individual prior to the methods of the invention. It will be appreciated that the activity and/or concentration of thymidine kinase (TK) may be measured. It is to be expected that TK protein levels correlate with TK activity levels. In one embodiment, TK activity (TKa) is measured. TKa can be measured by techniques well known in the art. It may be determined by ELISA, such as the DiviTum®TKa assay (Biovica, Sweden) in accordance with the manufacturer's instructions, which have previously been reported in Schwartz et al. (2003) J. Nucl. Med. 44 2027-2032, Nisman et al. (2013) Clinical Chemistry and Laboratory Medicine 51(2): 439-47, and Bagegni et al. (2017) Breast Cancer Res. 19(1): 123, and/or by a real time assay, such as those described in WO 2011/142719 and Stålhandske et al. (2013) Analytical Biochemistry 432; 155-164. TK concentration levels can also be measured by multiple techniques well known in the art, e.g. by immunoassays such as ELISA.

The sample provided in step (c) may, in an additional or alternative embodiment, be obtained from the individual 3-4 weeks after first treatment with the one or more immune checkpoint inhibitors.

The sample provided in step (c) may, in an additional or alternative embodiment, be obtained from the individual 7-28 days after first treatment with the one or more immune checkpoint inhibitors, or alternatively 14-28 days after first treatment with the one or more immune checkpoint inhibitors, or alternatively 21-28 days after first treatment with the one or more immune checkpoint inhibitors.

In an additional or alternative embodiment, the methods of the invention are for stratifying an individual or individuals having cancer for further treatment with one or more immune checkpoint inhibitors. In other words, the methods are used to determine whether an individual having cancer should receive further treatment with one or more immune checkpoint inhibitors.

In a further aspect of the invention there is provided a method for stratifying a patient having cancer for further treatment with one or more immune checkpoint inhibitors, the method comprising or consisting of the steps of:

i) performing the methods of the first aspect; and
ii) stratifying the patient for further treatment with one or more immune checkpoint inhibitors or an alternative cancer therapy based on the outcome of step (i), wherein the patient is stratified for further treatment with one or more immune checkpoint inhibitors when the activity and/or concentration of thymidine kinase (TK) measured in step (d) is increased compared to the activity and/or concentration of thymidine kinase (TK) measured in step (b)

optionally wherein the activity and/or concentration of thymidine kinase (TK) measured in step (b) and/or (d) is indicative of the prognosis of the individual if subsequently treated with one or more immune checkpoint inhibitors.

In one embodiment, the patient is stratified for further treatment with one or more immune checkpoint inhibitors. Alternatively, the patient may be stratified for no further ICI treatment. The patient may alternatively be stratified for treatment with an alternative non-ICI cancer therapy.

Alternative cancer treatments to immune checkpoint inhibitors will depend on the particular tumour type, however examples include surgery, chemotherapy, targeted therapy, immunotherapy (i.e. a different immunotherapy to immune checkpoint inhibitors), chemoimmunotherapy, radiotherapy and combinations thereof. For example, a physician may determine the most appropriate alternative cancer therapy by reference to the NCCN Cancer Treatment Guidelines for each individual tumour type where ICIs are approved (e.g. melanoma, lung, RCC, HNSCC, CRC, etc.), which can be found at https://www.nccn.org/guidelines/category_1.

The following embodiments are relevant to all aspects of the invention.

Preferably, the activity and/or concentration of thymidine kinase (TK) measured in step (d) is increased compared to the activity and/or concentration of thymidine kinase (TK) measured in step (b) by at least 8%, for example, at least 9%, at least 10%, at least 11%, at least 12%, at least 13%, at least 14%, at least 15%, at least 16%, at least 17%, at least 18%, at least 19%, at least 20%, at least 21%, at least 22%, at least 23%, at least 24%, at least 25%, at least 26%, at least 27%, at least 28%, at least 29%, at least 30%, at least 31%, at least 32%, at least 33%, at least 34%, at least 35%, at least 36%, at least 37%, at least 38%, at least 39%, at least 40%, at least 41%, at least 42%, at least 43%, at least 44%, at least 45%, at least 41%, at least 42%, at least 43%, at least 44%, at least 55%, at least 60%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, at least 100%, at least 125%, at least 150%, at least 175%, at least 200%, at least 225%, at least 250%, at least 275%, at least 300%, at least 350%, at least 400%, or at least 500%.

In an additional or alternative embodiment, the activity and/or concentration of thymidine kinase (TK) measured in step (d) is increased compared to the activity and/or concentration of thymidine kinase (TK) measured in step (b) by at least 8%.

In an additional or alternative embodiment, the activity and/or concentration of thymidine kinase (TK) measured in step (d) is increased compared to the activity and/or concentration of thymidine kinase (TK) measured in step (b) by at least 23%.

In an additional or alternative embodiment, the activity and/or concentration of thymidine kinase (TK) measured in step (d) is increased compared to the activity and/or concentration of thymidine kinase (TK) measured in step (b) by at least 25%.

In an additional or alternative embodiment, the activity and/or concentration of thymidine kinase (TK) measured in step (d) is increased compared to the activity and/or concentration of thymidine kinase (TK) measured in step (b) by at least 50%.

In additional or alternative embodiments, the activity and/or concentration of thymidine kinase (TK) measured in step (d) is increased by at least 100%, at least 110%, at least 120%, at least 130%, at least 140%, at least 150%, at least 160%, at least 170%, at least 180%, at least 190%, at least 200%, at least 250%, at least 300%, at least 350%, at least 400%, at least 450%, or at least 500% compared to the activity and/or concentration of thymidine kinase (TK) measured in step (b).

In an additional or alternative embodiment, the activity and/or concentration of thymidine kinase (TK) measured in step (d) is increased compared to the activity and/or concentration of thymidine kinase (TK) measured in step (b) by at least 191%.

By a "positive" prognosis, we include the individual having, or being expected to have, progression-free survival of at least 6 months, i.e. the cancer will not have progressed 6 months after the initial treatment with the ICI. The "positive" prognosis may include the individual having, or being expected to have, progression-free survival of at least 12, 18, or 24 months, i.e. the cancer will not have progressed 12, 18, or 24 months after the initial treatment with the ICI.

In an additional or alternative embodiment, a "positive" prognosis means progression-free survival or expected progression-free survival, of at least 1 year, or at least 2, 3, 4, 5, or 10 years, after the initial treatment with the ICI.

Progression-free survival (PFS) may be defined as the time from treatment start until the date of confirmed progression or the date of death or of the last follow-up.

In an additional or alternative embodiment, a "positive" prognosis means overall survival (OS), or expected overall survival, of at least 1 year, or at least 2, 3, 4, 5, or 10 years, after the initial treatment with the ICI. OS may be defined as the time from treatment start until the date of death or last follow-up.

In an additional or alternative embodiment, a "positive" prognosis means a complete response, a partial response, or stable disease. Preferably, the positive prognosis means a complete or partial response, most preferably a complete response. Response to treatment may be based on radiological investigations (CT, MRI, and/or positron emission (PET) CT tomography) evaluated by a radiologist and assessed according to the Response Evaluation Criteria in Solid Tumors (RECIST) 1.1 criteria [17].

By "stratified for further treatment with one or more immune checkpoint inhibitors", we include that the patient or individual having cancer is classified that they should receive treatment or further treatment with one or more immune checkpoint inhibitors. This may be the same or different ICI and may be alone or together with alternative cancer therapies.

In an additional or alternative embodiment, the prognosis of the individual if further treated with one or more checkpoint inhibitors is positive (for example the individual will, or is expected to, have progression free survival of more than 24 months), and/or the patient is stratified for further treatment with one or more immune checkpoint inhibitors, when the activity and/or concentration of thymidine kinase (TK) measured in step (d) is increased by at least 100% compared to the activity and/or concentration of thymidine kinase (TK) measured in step (b). Alternatively, the activity and/or concentration of thymidine kinase (TK) measured in step (d) is increased by at least 150%, at least 200%, at least 250%, at least 300%, at least 350%, at least 400%, at least 450%, or at least 500% compared to the activity and/or concentration of thymidine kinase (TK) measured in step (b).

In an additional or alternative embodiment, the prognosis of the individual if further treated with one or more immune checkpoint inhibitors is positive and/or the patient is stratified further treatment with one or more immune checkpoint inhibitors when the activity and/or concentration of thymidine kinase (TK) measured in step (b) is lower than a cut off value.

In an additional or alternative embodiment, the prognosis of the individual if further treated with one or more immune checkpoint inhibitors is negative (for example the individual would, or would be expected to, have progression free survival of less than 6 months) and/or the patient is stratified for no further treatment with one or more immune checkpoint inhibitors or treatment with an alternative cancer therapy to one or more immune checkpoint inhibitors, when the activity and/or concentration of thymidine kinase (TK) measured in step (b) is greater than or equal to a cut off value.

In an additional or alternative embodiment, the prognosis of the individual if further treated with one or more immune checkpoint inhibitors is positive (for example the individual will, or is expected to, have progression free survival of at least 6 months), and/or the patient is stratified further treatment with one or more immune checkpoint inhibitors, when the activity and/or concentration of thymidine kinase (TK) measured in step (b) is lower than a cut off value, and wherein the activity and/or concentration of thymidine kinase (TK) measured in step (d) is increased compared to the activity and/or concentration of thymidine kinase (TK) measured in step (b).

Preferably, the activity and/or concentration of thymidine kinase (TK) measured in step (d) is increased compared to the activity and/or concentration of thymidine kinase (TK) measured in step (b) by at least 8%, for example, at least 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 41%, 42%, 43%, 44%, 55%, 60%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, 125%, 150%, 175%, 200%, 225%, 250%, 275%, 300%, 350%, 400%, 500%.

In an additional or alternative embodiment, the activity and/or concentration of thymidine kinase (TK) measured in step (d) is increased compared to the activity and/or concentration of thymidine kinase (TK) measured in step (b) by at least 8%.

In an additional or alternative embodiment, the activity and/or concentration of thymidine kinase (TK) measured in step (d) is increased compared to the activity and/or concentration of thymidine kinase (TK) measured in step (b) by at least 23%.

In an additional or alternative embodiment, the activity and/or concentration of thymidine kinase (TK) measured in step (d) is increased compared to the activity and/or concentration of thymidine kinase (TK) measured in step (b) by at least 25%.

In an additional or alternative embodiment, the activity and/or concentration of thymidine kinase (TK) measured in step (d) is increased compared to the activity and/or concentration of thymidine kinase (TK) measured in step (b) by at least 50%.

In additional or alternative embodiments, the activity and/or concentration of thymidine kinase (TK) measured in step (d) is increased by at least 100%, at least 110%, at least 120%, at least 130%, at least 140%, at least 150%, at least 160%, at least 170%, at least 180%, at least 190%, at least 200%, at least 250%, at least 300%, at least 350%, at least 400%, at least 450%, or at least 500% compared to the activity and/or concentration of thymidine kinase (TK) measured in step (b).

In an additional or alternative embodiment, the activity and/or concentration of thymidine kinase (TK) measured in step (d) is increased compared to the activity and/or concentration of thymidine kinase (TK) measured in step (b) by at least 191%.

In an additional or alternative embodiment, the prognosis of the individual if further treated with one or more immune checkpoint inhibitors is positive (for example the individual will, or is expected to, have progression free survival of more than 24 months), and/or the patient is stratified for further treatment with one or more immune checkpoint inhibitors, when the activity and/or concentration of thymidine kinase (TK) measured in step (b) is lower than a cut off value, and wherein the activity and/or concentration of thymidine kinase (TK) measured in step (d) is increased by at least 100% compared to the activity and/or concentration of thymidine kinase (TK) measured in step (b).

It will be appreciated that the percentage increase value may be optimised depending on the cancer or tumour type. A skilled person in this field would be able to modify a percentage increase value as a matter of routine based on the information described herein for melanoma.

In additional or alternative embodiments, the prognosis of the individual if further treated with one or more immune checkpoint inhibitors is negative (for example the individual would have or would be expected to have, progression free survival of less than 6 months), and/or the patient is stratified for no further treatment with one or more immune checkpoint inhibitors or treatment with an alternative cancer therapy to one or more immune checkpoint inhibitors, when the activity and/or concentration of thymidine kinase (TK) measured in step (b) is greater than or equal to a cut off value, and wherein the activity and/or concentration of thymidine kinase (TK) measured in step (d) is not increased compared to the activity and/or concentration of thymidine kinase (TK) measured in step (b).

In additional or alternative embodiments of the methods of the invention, it is the activity of thymidine kinase (TKa) that is measured and the cut off value is selected from between 549 and 1021 Du/L, for example between 628 and 942 Du/L, for example between 707 and 864 Du/L.

In additional or alternative embodiments of the methods of the invention, it is the activity of thymidine kinase (TKa) that is measured and the cut off value is selected from between 385 and 715 DuA, for example between 440 and 660 DuA, for example between 495 and 605 DuA. The relationship between Du/L and Du/A is described below.

In an additional or alternative embodiment of the methods of the invention, the cut off value is 785 Du/L (+/- 20%). In an additional or alternative embodiment of the methods of the invention, the cut off value is 785 Du/L (+/- 10%). 10% reflects the normal assay variance.

In an additional or alternative embodiment of the methods of the invention, the cut off value is 550 DuA (+/- 20%). In an additional or alternative embodiment of the methods of the invention, the cut off value is 550 DuA (+/- 10%). 10% reflects the normal assay variance.

In additional or alternative embodiments of the methods of the invention, it is the activity of thymidine kinase (TKa) that is measured and the cut off value is selected from between 40 and 75 Du/L, for example between 42 and 72 Du/L, for example between 49 and 72 Du/L, for example between 42 and 60 Du/L, for example between 49 and 60 Du/L, for example between 58 and 60 Du/L, or preferably 60 Du/L. In one embodiment the cut off is 60 Du/L. In an alternative embodiment the cut off is 58 Du/L. In alternative or additional embodiments, the cut off value may be 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, or 75 Du/L.

It will be appreciated that a pre-treatment cut off value may be optimised depending on the cancer or tumour type and the number or prior therapies that a patient has already received. A skilled person in this field would be able to modify a cut off value as a matter of routine based on the information described herein.

By Du/L we mean DiviTum® units per litre in accordance with the DiviTum®TKa assay (Biovica, Sweden) and the manufacturer's instructions, which have previously been reported in Schwartz et al. (2003) J. Nucl. Med. 44 2027-2032, Nisman et al. (2013) Clinical Chemistry and Laboratory Medicine 51(2): 439-47, and Bagegni et al. (2017) Breast Cancer Res. 19(1): 123.

TKa may also be measured with the DiviTum®TKa assay by the unit DuA. For the unit Du/L, a recombinant thymidine kinase protein is used for assigning Du/L values for a panel of "gold standard sera" and calibrators. A measurement of 1 Du/L is the catalytic activity obtained from a sample with the concentration of 1 pg of recombinant thymidine kinase (TK) per ml. An optimized DiviTum®TKa assay with a new calibration concept to better match the assay principle of measuring TK activity, and a new unit (DuA) better reflecting TK activity, has also been developed. The conversion equation between Du/L and DuA is as follows:

$$DuA = 134 + 0.53(Du/L). \text{ Coefficient of determination } (R^2) = 0.93$$

In an additional or alternative embodiment, the method further comprises the steps of:

(e) providing one or more control samples; and
(f) measuring the activity and/or concentration of thymidine kinase (TK) in the sample provided in step (e);

wherein the prognosis of the individual if further treated with one or more immune checkpoint inhibitors is determined by comparing the measurement in step (f) with the measurement in step (b) and/or step (d).

The one or more control sample may be obtained from an individual having cancer before subsequently undergoing successful treatment with one or more immune checkpoint inhibitors.

By successful treatment we include arrest, non-progression, and/or regression in tumour growth. This may be evaluated through imaging technologies, e.g. a gold standard evaluation tool. Successful treatment may be evaluated through the RECIST criteria (see reference 17). Response Evaluation Criteria In Solid Tumours or (RECIST) refers to a set of published rules used to assess tumour burden in order to provide an objective assessment of response to therapy.

Stable disease, partial response, complete response, progression free survival and overall survival are endpoints where it is considered successful treatment. Successful treatment may be defined in the same way as a positive prognosis is defined above. For example, successful treatment may be considered to be progression-free survival of at least 24 months, i.e. the cancer will not have progressed 24 months after the initial treatment with the ICI.

In an additional or alternative embodiment, the prognosis of the individual if further treated with one or more immune checkpoint inhibitors is positive and/or the patient is stratified for further treatment with one or more immune checkpoint inhibitors when the activity and/or concentration of thymidine kinase (TK) measured in step (b) corresponds to or is lower than the activity and/or concentration of thymidine kinase (TK) measured in step (f).

By "corresponds to" we include that the activity and/or concentration of thymidine kinase (TK) is identical to that of a positive control sample; or closer to that of one or more positive control sample than to one or more negative control sample (or to predefined reference values representing the same).

Alternatively or additionally, by "corresponds to" we include that the activity and/or concentration correlates with the amount in the control sample in a statistically significant manner. By "correlates with the amount in the control sample in a statistically significant manner" we mean or include that the presence or amount in the test sample correlates with the that of the control sample with a $p$-value of $\leq 0.05$, for example, $\leq 0.04$, $\leq 0.03$, $\leq 0.02$, $\leq 0.01$, $\leq 0.005$, $\leq 0.004$, $\leq 0.003$, $\leq 0.002$, $\leq 0.001$, $\leq 0.0005$ or $\leq 0.0001$.

In an additional or alternative embodiment, the one or more control sample may be obtained from an individual having cancer before subsequently undergoing unsuccessful treatment with one or more immune checkpoint inhibitors.

In an additional or alternative embodiment, the prognosis of the individual if further treated with one or more immune checkpoint inhibitors is negative and/or the patient is stratified for no further treatment with one or more immune checkpoint inhibitors or treatment with an alternative cancer therapy to one or more immune checkpoint inhibitors when the activity and/or concentration of thymidine kinase (TK) measured in step (b) is higher than the activity and/or concentration of thymidine kinase (TK) measured in step (f).

In an additional or alternative embodiment of the methods of the invention, the method further comprises the step of:

g) measuring the activity and/or concentration of lactate dehydrogenase (LDH) in the sample provided in step (a); wherein the activity and/or concentration of LDH measured in step (g) is additionally indicative of the prognosis of the individual if subsequently treated with one or more immune checkpoint inhibitors.

In an additional or alternative embodiment, the prognosis of the individual if treated or further treated with one or more immune checkpoint inhibitors is positive (for example the individual will have progression free survival of more than 24 months), and/or the patient is stratified for treatment or further treatment with one or more immune checkpoint inhibitors, when the activity and/or concentration of LDH measured in step (g) is considered normal, i.e. not elevated above normal levels.

In an additional or alternative embodiment, the prognosis of the individual if treated or further treated with one or more immune checkpoint inhibitors is positive (for example the individual will have progression free survival of more than 24 months), and/or the patient is stratified for treatment or further treatment with one or more immune checkpoint inhibitors, when the activity and/or concentration of LDH measured in step (g) is lower than a cut off value.

In an additional or alternative embodiment it is the activity of LDH which is measured and the cut off value is selected from between 2 and 6 mikrokat/L, for example between 3 and 5 mikrokat/L, or for this example is 4 mikrokat/L.

It will be appreciated that the cut off value may be optimised, e.g. depending on the cancer or tumour type of the individual. This may be achieved as a matter of routine by a skilled person in this field based on the information described herein.

In an additional or alternative embodiment, the cancer is a cancer approved for treatment with an immune checkpoint inhibitor. For example, we include patients with cancers that a regulatory agency such as the FDA has approved for treatment with an immune checkpoint inhibitor. Twomey and Zhang (2021) The AAPS Journal 23:39 provides a review of FDA-approved immune checkpoint inhibitors.

In one embodiment of any of the methods of the invention, the cancer may be one or more of: melanoma, MSI-H/dMMR colorectal cancer, pleural mesothelioma, triple-negative breast cancer, cutaneous squamous cell carcinoma, colorectal cancer, Bacillus Calmette-Guérin, bladder cancer, endometrial carcinoma, esophageal squamous cell carcinoma, small cell lung cancer, renal cell carcinoma, Merkel cell carcinoma, hepatocellular carcinoma, primary mediastinal large B cell lymphoma, cervical cancer, gastric cancer, urothelial carcinoma, classical Hodgkin's lymphoma, head and neck squamous cell carcinoma, non-small cell lung cancer.

In one embodiment of any of the methods of the invention, the cancer is selected from the group comprising: melanoma; breast cancer; and/or ovarian cancer. The cancer may be metastatic.

In one embodiment of any of the methods of the invention, the cancer is melanoma and/or breast cancer.

In one embodiment of any of the methods of the invention, the cancer is melanoma. The melanoma may be metastatic melanoma.

In one embodiment of any of the methods of the invention, the cancer is breast cancer. The breast cancer may be metastatic breast cancer. The breast cancer may be hormone receptor-positive.

In one embodiment of any of the methods of the invention, the cancer is ovarian cancer. The ovarian cancer may be metastatic ovarian cancer.

In one embodiment of any of the methods of the invention, the one or more immune checkpoint inhibitors are selected from the group consisting of: CTLA-4 inhibitor, PD-1 inhibitor, PD-L1 inhibitor, LAG-3 inhibitor.

In an additional or alternative embodiment, the one or more checkpoint inhibitors are anti-CTLA-4 and/or anti-PD-1.

In an additional or alternative embodiment, the one or more checkpoint inhibitors are selected from the group consisting of: nivolumab, pembrolizumab, ipilimumab, and/or spartalizumab.

In one embodiment of any of the methods of the invention the individual or patient is a human.

In a further embodiment of any of the methods of the invention, the method is performed *in vitro* or *ex vivo.*

In a further embodiment of any of methods of the invention, the sample is a blood, serum or plasma sample. Preferably, the sample is a serum or plasma sample.

In a further aspect of the invention there is provided one or more immune checkpoint inhibitors for use in treating cancer in a patient, wherein the patient has been selected for further treatment on the basis of their TK activity and/or concentration prior to treatment with one or more immune checkpoint inhibitors and, also their TK activity and/or concentration measured 1-4 weeks after first treatment with the one or more immune checkpoint inhibitors, wherein the patient is selected for further treatment due to having a serum or plasma TK activity and/or concentration 1-4 weeks after treatment with an ICI inhibitor which is increased compared to their serum or plasma TK activity and/or concentration prior to ICI treatment.

In an embodiment of the preceding aspects, the individual is selected for further ICI treatment due to having a TK activity and/or concentration prior to ICI treatment which is lower than a cut off value, optionally wherein the cut off value is a particular cut off value selected from those values described in the earlier aspects of the invention.

In an additional embodiment of the preceding aspects, the individual is selected for further ICI treatment due to having a TK activity and/or concentration 1-4 weeks, optionally 3-4 weeks, after treatment with an ICI inhibitor which is increased compared to their TK activity and/or concentration prior to ICI treatment, wherein the increase is at least a particular percentage increase selected from those values described in the earlier aspects of the invention, for example at least a 100% increase.

In an additional embodiment of the preceding aspects, the individual is selected for further ICI treatment due to: (i) having a TK activity and/or concentration prior to ICI treatment which is lower than a cut off value, optionally wherein the cut off value is a particular cut off value selected from those values described in the earlier aspects of the invention; and (ii) having a TK activity and/or concentration 1-4 weeks, optionally 3-4 weeks, after treatment with an ICI inhibitor which is increased compared to their TK activity and/or concentration prior to ICI treatment, optionally wherein the increase is at least a particular percentage increase selected from those values described in the earlier aspects of the invention, for example at least a 100% increase.

In a further embodiment of the preceding aspects, the individual is additionally selected for ICI treatment due to having an LDH activity and/or concentration prior to ICI treatment which is lower than a cut off value or is considered normal, optionally wherein the cut off value is a particular cut off value selected from those values described in the earlier aspects of the invention.

In a further embodiment of the preceding aspects, the TK and/or LDH activity and/or concentration is measured in a blood (e.g. unfractionated blood), plasma, serum, tissue fluid, and/or urine sample.

In a further embodiment of the preceding aspects, the TK and/or LDH activity and/or concentration is measured in a serum or plasma sample.

In a further aspect of the invention there is provided the use of thymidine kinase as a prognostic biomarker for individuals having cancer being considered for treatment with or currently treated with immune checkpoint inhibitors.

In a further aspect of the invention there is provided the use of thymidine kinase for stratifying individuals having cancer for treatment or further treatment with immune checkpoint inhibitors.

Preferences and options for a given aspect, feature or parameter of the invention should, unless the context indicates otherwise, be regarded as having been disclosed in combination with any and all preferences and options for all other aspects, features and parameters of the invention. For example, preferences, options, and embodiments described in relation to the methods of determining prognosis also apply equally to the methods of stratifying patients, methods of treatment, and uses subsequently described.

The listing or discussion of an apparently prior-published document in this specification should not necessarily be taken as an acknowledgement that the document is part of the state of the art or is common general knowledge.

Preferred, non-limiting examples which embody certain aspects of the invention will now be described, with reference to the following figures and examples:

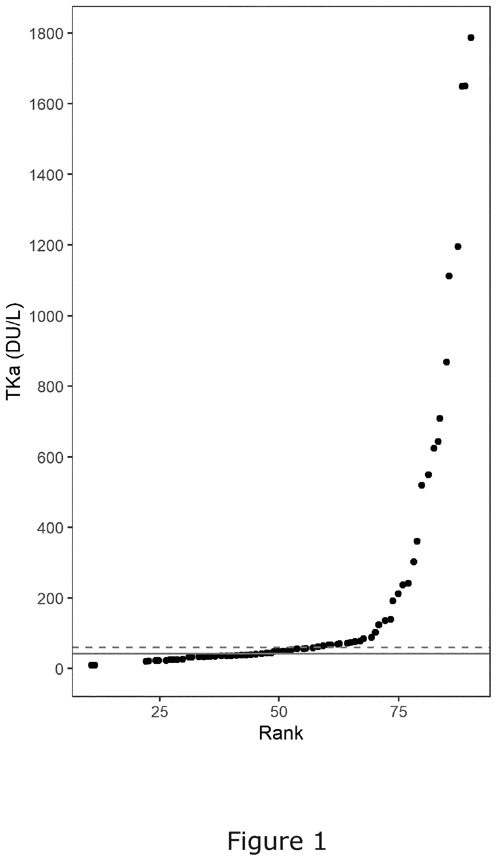

**Figure 1: Determining the cut-off for thymidine kinase 1 activity (TKa) in the plasma of metastatic melanoma**

**patients treated with immune checkpoint inhibitors.** Rank (order) of TKa in DiviTum® units per liter (Du/L) is shown on the x-axis and the TKa value is shown on the y-axis. When determining the cut-off value for TKa, the most optimal cut-off was considered as the value closest to the median that ensured a differentiation in the TKa value with sufficient numbers of observations in each group. The median TKa at baseline was 42 Du/L. This median (solid line) gave a cut-off that divided patients with very similar TKa values into different groups. A cut-off of 60 Du/L (dotted line) ensured more differentiation in TKa as well as enough observations in both groups.

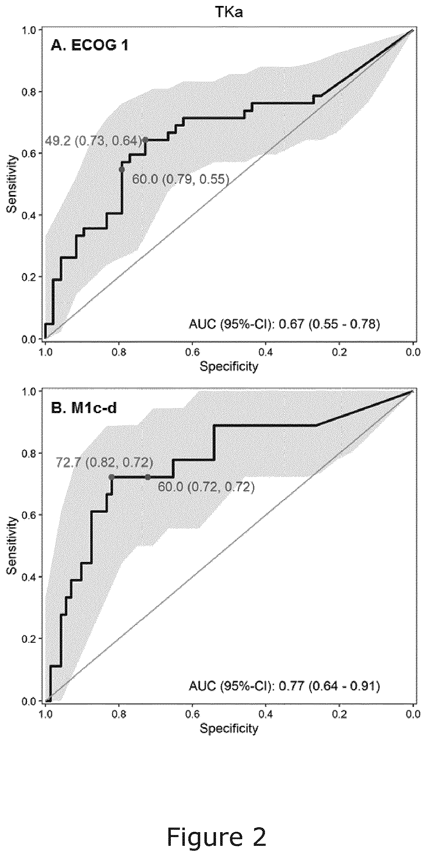

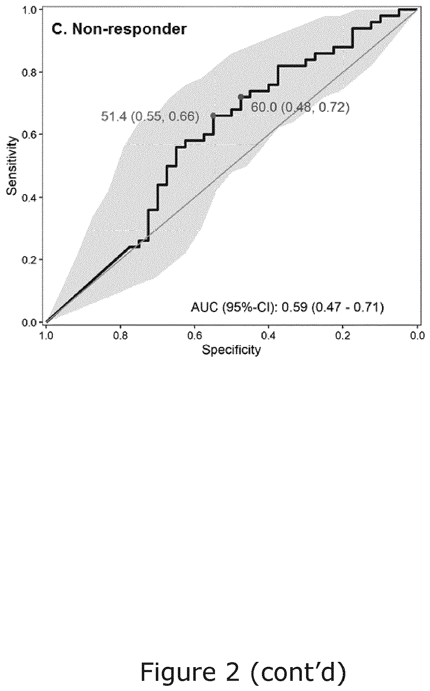

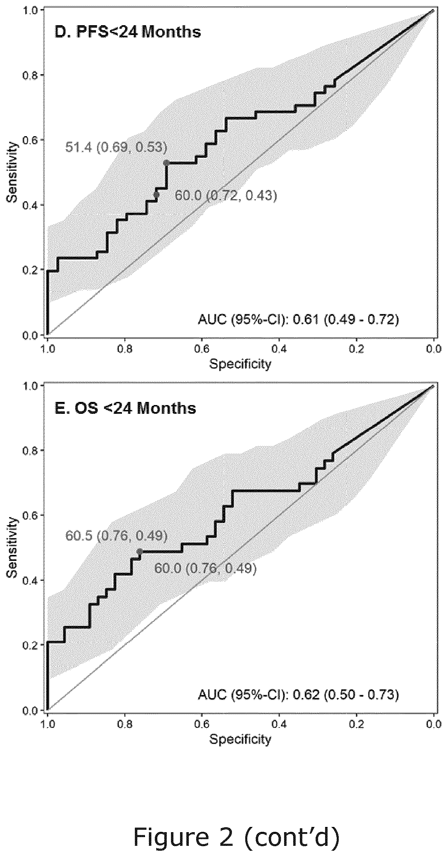

**Figure 2: ROC curve analysis with the most optimal TKa cut-offs for the sensitivity and specificity in predicting: A. Performance stage (ECOG $\geq$1 vs. ECOG 0) B. Tumour stage (M1c-d vs. M1a-b,),) C. Response (SD and PD vs. CR and PR), D. Progression-free survival (shorter or longer than 24 months) and E. Overall survival (shorter or longer than 24 months).** The optimal TKa cut-off for each of the analysis is shown for each of the analyses (numbers above the trend line) and the TKa of 60.0 that was selected as a cut-off for the comparative analyses in the study is shown below the trend line.

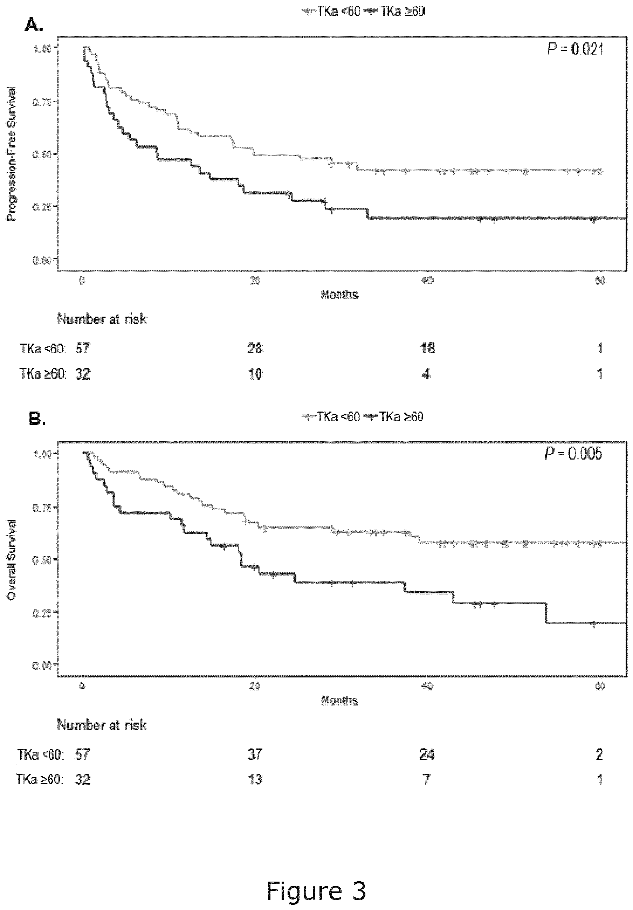

**Figure 3: Kaplan-Meier curves for survival in melanoma patients with high (>60 Du/L) or low thymidine kinase 1 activity (TKa) in plasma before starting a treatment with immune checkpoint inhibitors.** (A). The median progression-free survival was, in patients with low TKa, 19.9 months (95% CI, 11.0 to not reached) and in patients with high TKa, 12.6 months (95% CI, 3.6 to 28.3) (p = 0.021). (B). The median overall survival was not reached in patients with low TKa (>60 months, 95% CI, 38.0 to not reached), and in the patients with high TKa it was 18.5 (p = 0.005).

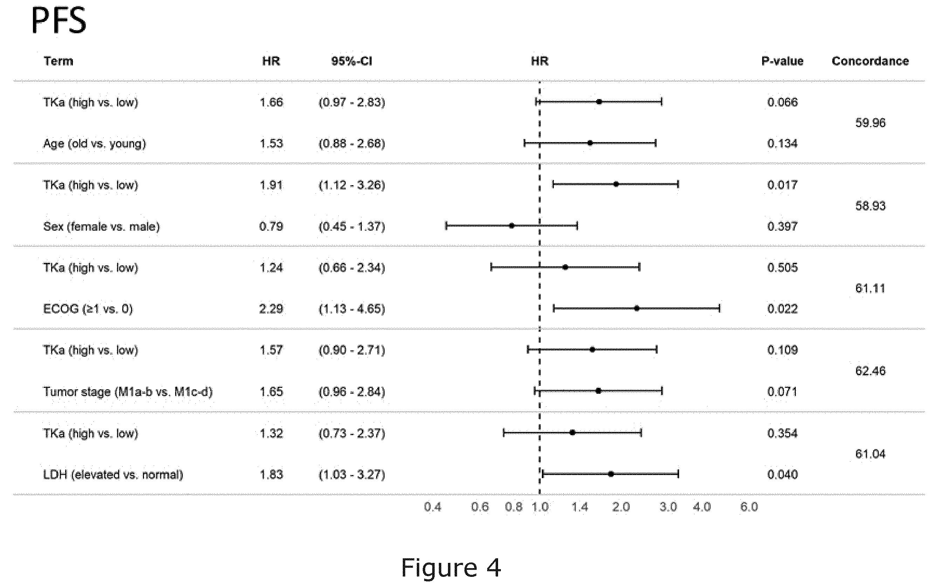

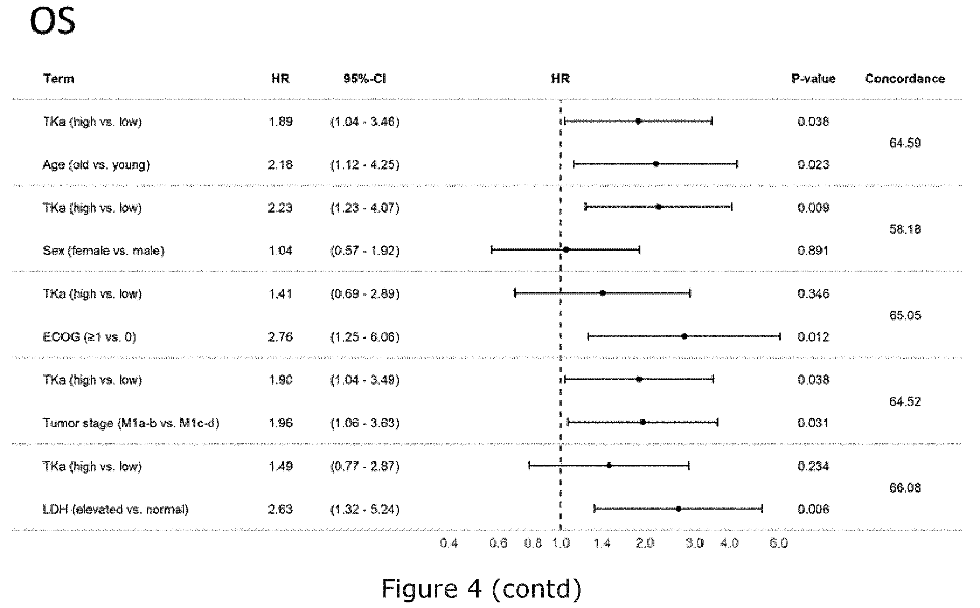

**Figure 4: Bivariable regression with pairwise analyses of TKa together with one other variable for A. progression-free survival (PFS) and B. overall survival (OS) in metastatic melanoma patients treated with immune checkpoint inhibitors.**

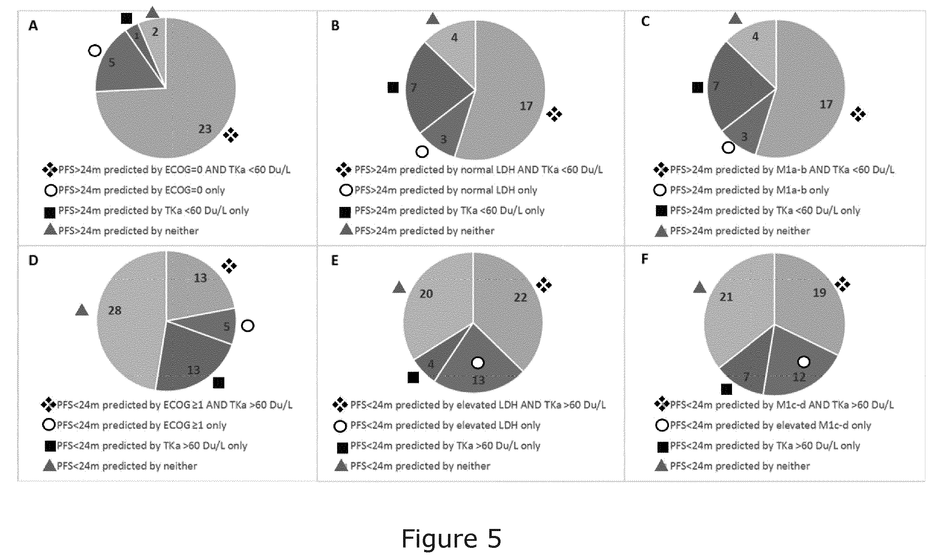

**Figure 5: Pie charts showing the number of patients with longer (>24 months, (A-C)) or shorter (<24 months, (D-F)) progression-free survival (PFS).** Each pie chart shows if longer or shorter PFS was correctly predicted by the TKa level together with one other baseline variable (ECOG, LDH, or M stage). For example (B) shows that in 17 patients, long PFS was predicted by both low TKa and normal LDH at baseline (correctly predicted by both variables, diamond symbol), in 3 patients long PFS was predicted only by normal LDH, as TKa was elevated (correctly predicted only by LDH, circle symbol), in 7 patients long PFS was predicated only by low TKa, as LDH level was elevated (correctly predicted only by TKa, square symbol), and in 4 patients long PFS was predicted by neither variable, as both LDH and TKa levels were high (correctly predicted by none, triangle symbol).

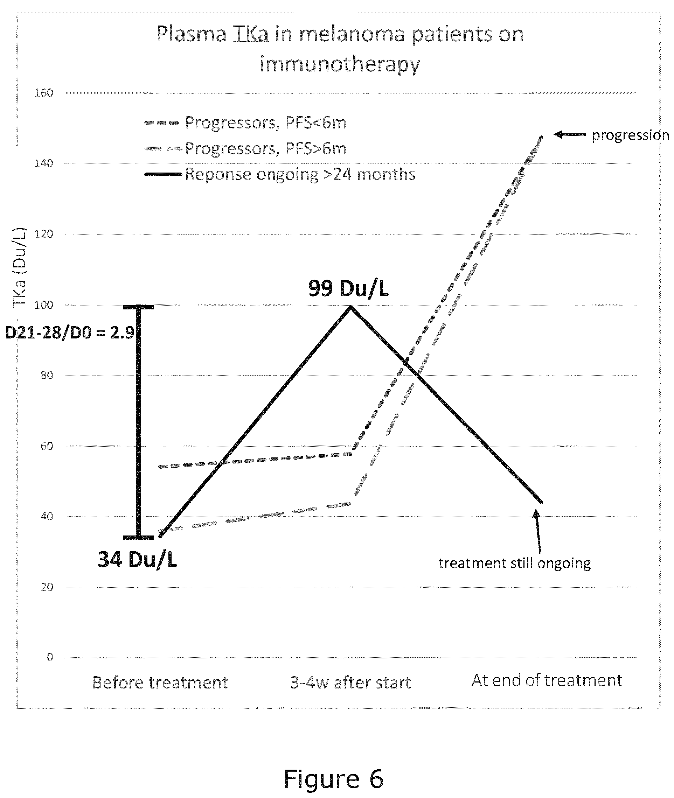

**Figure 6: Plasma TKa in melanoma patients on immunotherapy.** Plot shows the median TKa values in Du/L at 3 separate timepoints (pre-treatment, 3-4 weeks after treatment, and at end of treatment or at 24 months if patient was still on treatment). Patients were stratified according to their progression free survival time (PFS<6 months, PFS> 6 months, or response still ongoing at 24 months). Patients with the shortest PFS times of less than 6 months had the highest pre-treatment levels of TKa and little to no on-treatment increase of TKa. Patients who had a PFS time of between 6-24 months, had a low pre-treatment TKa but a less than 2-fold on-treatment increase of TKa. Patients who had a response to treatment lasting longer than 24 months, had a low pre-treatment TKa level AND a 2-fold or greater on-treatment increase of TKa.

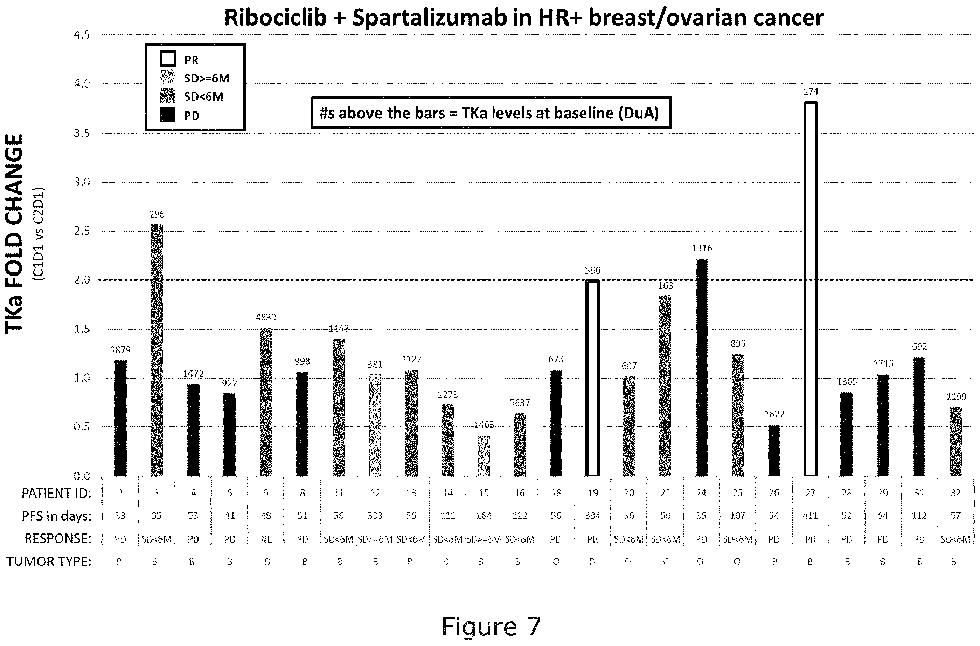

**Figure 7: Correlation between TK activity levels and outcome of HR+ breast cancer and ovarian cancer patients treated with ribociclib and the PD-1 inhibitor spartalizumab.** Individual patient ID numbers are listed on the horizontal axis. Height of each bar represents the fold increase in TKa levels from cycle 1 day 1 (pre-treatment) to cycle 2 day 1 (28 days after first treatment) for the indicated patient. The numbers above each bar are that patient's raw baseline (pre-treatment) TKa value. The bar shading indicates the best overall response as assessed by RECIST 1.1 criteria where:

Black/PR=partial response;
Dark Grey/SD>6M = stable disease for greater than or equal to 6 months;
Light Grey/SD<6M=stable disease for less than 6 months;
White/PD= progressive disease;
NE = not evaluable.

Best overall response is also indicated in the horizontal axis along with the progression free survival (PFS) time in days and the tumor type for each patient where:

B=HR+ Her2- metastatic breast cancer
O=metastatic epithelial ovarian cancer

## EXAMPLE 1

### Summary and Introduction

Immune checkpoint inhibitors (ICI) are effective in fractions of patients with disseminated melanoma. The most clinically developed ICIs are those targeting programmed cell death-1 (PD-1), programmed cell death ligand-1 (PD-L1), cytotoxic T-lymphocyte antigen-4 (CTLA-4), and lymphocyte activation gene-3 (LAG-3). Antibody drugs targeting these immune checkpoints remove the brakes on T-cell activity, which enables the activation and subsequent proliferation of tumour-reactive T cells which are then able to mount an effective antitumor response. Studies have shown significant increases in the number of T-cells following the first dose of ICI drugs. The level of this increase in T-cells correlates with tumour response and patient outcome [28]. The DiviTum®TKa assay described herein is able to detect this proliferative burst of T-cells in patients within the first month of ICI treatment.

In recent years, effective ICI regimens with CTLA-4 and PD-1 blocking antibodies have emerged for the treatment of melanoma [1-6]. These treatments have revolutionised the melanoma oncology field, but a considerable fraction of melanoma patients do not respond or get lasting effects from these treatments. Significant toxicity can also occur from the treatments, that, in addition, are expensive. It is therefore important to increase knowledge of predictive factors and their efficacy in different patient groups.

Thymidine kinase 1 (TK) is a cytosolic enzyme, a phosphotransferase that plays a pivotal role in DNA synthesis and repair [7]. TK has a key function in DNA synthesis and cell division as it is part of the reaction chain to introduce thymidine into the DNA strand [7]. Dividing cells release TK during mitotic exit, and TK can thus be detected in the blood. Further, elevated TK enzyme activity has been measured in blood samples from cancer patients and is associated with tumour proliferation and tumour burden [8]. Circulating levels of TKa, measured with the DiviTum® assay, have been shown to be associated with disease stage, prognosis, and treatment efficacy in several cancer types, including breast, lung, pancreatic, and renal cell cancer [9-15]. Pre-treatment levels of TKa can reflect both the rate of tumour cell proliferation as well as total disease burden, with high levels of TKa indicative of actively proliferating tumour(s) and/or a large disease burden (in both tumour size and number), and low levels of TKa indicative of slow growing, indolent tumours and a lower disease burden (smaller and fewer total tumours).

The inventors analysed for the first time the plasma activity of TK, an enzyme involved in DNA synthesis and repair, as a biomarker in melanoma patients. TK activity (TKa) levels in metastatic melanoma patients were measured before starting ICI treatment and correlated with baseline clinical characteristics, treatment response, and survival.

The inventors found that high TKa levels in melanoma patients were associated with poor baseline factors, such as poor performance status, high plasma lactate dehydrogenase levels, and advanced tumour stage. High TKa levels were also associated with a poor efficacy of immune checkpoint inhibitors. TKa has therefore been identified by the inventors as a novel prognostic and predictive marker in cancer.

### Materials and Methods

#### Patients and Plasma Samples:

Plasma samples were collected from patients with unresectable metastatic cutaneous melanoma, treated with ICI (anti-CTLA-4 and/or anti-PD-1) at the Department of Oncology, Karolinska University Hospital, Stockholm, Sweden in the years 2012-2019. The treatments were administrated according to standard ICI regimens and dosage approved for the treatment of metastatic melanoma. Blood samples were taken from the patients within 5 days prior to treatment start. The blood samples were collected in EDTA tubes and centrifuged at 1500x g for 10 min, and the separated plasma was centrifuged at 2400x g for 15 min and frozen at -70°C within 1 hour of processing. The baseline clinical data included age at treatment start, gender, Eastern Cooperative Oncology Group (ECOG) performance status, baseline tumour stage according to the American Joint Committee (AJCC) on Cancer, Eighth Edition [16], number of affected organs, baseline lactate dehydrogenase (LDH) levels, previous lines of treatment, and ICI regime received after TKa sampling. The study was conducted in accordance with Good Clinical Practice, with informed consent from all patients, and was approved by the Stockholm Regional Ethics Committee.

#### TK Activity Level Analysis:

Plasma TKa levels were determined using the DiviTum®TKa assay (Biovica, Sweden) in accordance with the manufacturer's instructions, which have previously been reported [7]. DiviTum®TKa is a refined ELISA-based test reflecting cell proliferation rate by measuring TKa in serum, plasma, or cells. In summary, plasma was mixed with the reaction mixture in a 96-well ELISA plate, and bromodeoxyuridine (BrdU) monophosphate was generated by TK reaction, phosphorylated to BrdU triphosphate, and incorporated into a synthetic DNA strand. An anti-BrdU monoclonal antibody conjugated to the enzyme alkaline phosphatase and a chromogenic substrate were used to detect BrdU incorporation. The absorbance readings were converted using standards with known TKa values (working range from 20 to 4000 Du/L). The lower limit of detection of the assay was set at 20 Du/L, and all values below the threshold were reported as <20 Du/L. All plasma TKa analyses were performed in the Biovica laboratory (Uppsala, Sweden) where the personnel were blinded to patient and tumour data. The samples were measured in duplicate and fulfilled the Coefficient of Variation (CV) criteria of the DiviTum®TKa assay (CV < 20%). An optimized DiviTum®TKa assay for measuring TKa is a CE-IVD-labelled assay and has also been submitted to the FDA in a 510 (k) application that is awaiting approval. As mentioned above, the optimized DiviTum®TKa assay has a new calibration concept to better match the assay principle of measuring TK activity, and a new unit (DuA) better reflecting TK activity. The conversion equation between Du/L and DuA is as follows:

$$DuA = 134+0.53(Du/L). \text{ Coefficient of determination } (R^2) = 0.93$$

**Follow-Up:**

Routine follow-up after initiating the ICI treatment included monthly clinical assessments and radiological evaluations every third month. The patients had a minimum follow-up of 24 months. The patients were grouped based on TKa levels in plasma at baseline (low or high) and followed for treatment response, progression-free survival (PFS), and overall survival (OS). Best response to treatment was based on radiological investigations (CT, MRI, and/or positron emission (PET) CT tomography) evaluated by a radiologist and assessed according to the Response Evaluation Criteria in Solid Tumours (RECIST) 1.1 criteria [17]. Response rate (RR) was defined as the frequency of patients with partial (PR) or complete responses (CR) as the best response. Disease control rate (DCR) was defined as the frequency of patients with PR, CR, or stable disease (SD) as the best response after at least three months of treatment. PFS was defined as the time from treatment start until the date of confirmed progression or the date of death or of the last follow-up. OS was defined as the time from treatment start until the date of death or last follow-up.

**Statistical Methods:**

A receiver operating characteristic (ROC) analysis was performed to investigate TKa cut-offs with the most optimal sensitivity and specificity to predict tumour stage, performance stage, response, and survival. Baseline characteristics and treatment responses were compared with the Chi-square test for categorical variables and the Student's t-test for continuous variables. p values <0.05 were deemed statistically significant. The time to event outcomes for PFS and OS were analysed with Kaplan-Meier curves and Cox proportional hazards regression. Median PFS and OS with 95% confidence intervals (CI) were assessed. Univariable, bivariable, and multivariable models for Cox regression were used to assess each predictor's association with PFS and OS. Hazard ratios (HR) and corresponding two-sided 95% CI were estimated. Statistical analyses were performed with R Version 4.1.1. Concordance is a measure of the model's predicative accuracy measured as the proportion of all evaluable pairs of subjects where the model correctly predicts a higher risk for the individual in the pair with the worst outcome.

Results

**Baseline Characteristics:**

A total of 90 patients with metastatic melanoma were included in the study. The median pre-treatment plasma TKa level was 42 Du/L (range <20-1787 Du/L). There were no significant differences in TKa levels related to the age or the sex of the patients (Table 1). However, a significantly higher plasma TKa was found in patients with ECOG performance status ≥1 vs. 0-1 (p = 0.003), with M1c-M1d vs. M1a-M1b disease (p = 0.015), or with elevated vs. non-elevated LDH (p < 0.001). The TKa levels were higher in patients who were previously treated or had more than three affected organs, but here the difference in TKa was not significantly different. In patients with M1b-d disease, the TKa level was compared depending on if the patients had metastatic spread to a certain organ or not. This analysis was done separately from that on M1a patients in an attempt to address if the TKa level was affected by the spread to a specific organ, rather than assessing the tumour burden (as M1a patients typically have substantially less tumour burden). To conclude, no significant differences were observed regarding which organ was affected.

**Determining the Cut-Off for TKa:**

As TKa has not been studied in melanoma before, an essential aim was to determine a suitable cut-off and then compare patients with high or low plasma TKa levels. The median TKa at baseline (42 Du/L) gave a cut-off that divided patients with very similar TKa levels into different groups (Figure 1). In that sense, 60 Du/L was considered as a more suitable cut-off, since it ensured more differentiation in TKa levels and gave sufficient numbers of patients with high or low TKa levels. In a next step, ROC analyses were performed to determine TKa cut-offs with the most optimal sensitivity and specificity in predicting baseline characteristics and outcomes (Figure 2). The ROC analyses demonstrated that TKa cut-offs between 49 and 72 achieved the highest sensitivity and specificity to predict tumour stage, performance stage, ICI response, PFS and OS at 24 months (the median of these cut-offs was 58). The ROC analyses hence further supported the use of 60 Du/L as a reasonable cut-off.

**Characteristics and Outcomes of Patients with High or Low TKa:**

In melanoma patients with high ($\geq$60 Du/L) or low plasma TKa levels, there were no significant differences in the age, sex, or the tumour BRAF mutation status (Table 2). However, a high TKa level was significantly associated with ECOG performance status $\geq$1 (p < 0.001), M1c or M1d disease (p = 0.002), $\geq$3 affected organs (p = 0.031), elevated LDH (p < 0.001), and a higher median LDH (p < 0.001). In patients with high or low TKa, no significant differences were seen with respect to whether previous treatment lines had been received or to the ICI regime chosen for the patient. The majority of the patients were treated in first line with PD-1 inhibitor monotherapy (nivolumab or pembrolizumab). A smaller portion received a single CTLA-4 inhibitor (ipilimumab) or a CTLA-4 and PD-1 inhibitor combination (ipilimumab and nivolumab). Although there was not a statistically significant difference, more patients in the TKa-low group received combination immunotherapy (n = 6) compared to the TKa-high group (n = 0). A plausible explanation is that the better performance status and somewhat younger age of the TKa-low patients resulted in the fact that they more often were assessed and found to be able to tolerate the more toxic combination therapy.

The RR was significantly higher for patients with low TKa (63.2%) than for those with high TKa (30.3%) (p = 0.022) (Table 3). The rate of complete response was also higher for the TKa-low group (33.3%) than for the TKa-high group (6.0%) (p = 0.016). The DCR was also higher for patients with low (80.7%) vs. those with high (54.3%) TKa (p = 0.022). No difference was seen related to why the treatment was ended (progressive disease, adequate response, or toxicity) (Table 3).

The median PFS was 19.9 months (95% CI, 11.0 to not reached) in patients with low TKa and 12.6 months (95% CI, 3.6 to 28.3) in patients with high TKa (p = 0.021) (Figure 3). The median OS was not reached (>60 months, 95% CI, 38.0 to not reached) in patients with low TKa and was 18.5 months (95% CI, 11.7 to not reached) in patients with high TKa, (p = 0.005).

The univariate Cox regression analysis showed significantly worse PFS and OS for patients with baseline ECOG performance status $\geq$1, M1c or M1d disease, elevated LDH, and a high TKa (Table 4). For TKa the HR for PFS was 1.83 (95% CI, 1.08-3.08), p = 0.024, and that for OS was 2.25 (95% CI, 1.25-4.05), p = 0.007. In the multivariate analysis, TKa was not significant for PFS or OS. A high degree of multicollinearity amongst the analysed variables was identified as a factor that resulted in that the HR for many of the variables that were significant in the univariate model, were not significant in the multivariate model.

To evaluate how the TKa was affected by each of the covariates, a bivariate regression analysis was performed where TKa was analysed pairwise with one other baseline factor (Figure 4). As for the PFS, TKa was only independent of the patients' sex.

Further, in the bivariable analysis for OS, TKA was independent of age, sex, and tumour stage. Figure 5 shows the number of patients with longer (>24 months, Figure 5A-C) or shorter (<24 months, Figure 5D-F) PFS; each chart shows if longer or shorter PFS was correctly predicted by the TKa level together with one other baseline variable (ECOG, LDH, or M stage). The charts show that, although there was a substantial overlap of TKa and the other variables (diamond symbols), in a fraction of patients, low or high TKa levels alone were associated with long or short PFS, respectively (square symbols).

As part of the same study, TKa levels were also measured for 58 patients during immunotherapy treatment (at 3-4 weeks following start of treatment, and at the end of treatment). The results are shown in Table 5 and Figure 6. This shows that patients with the shortest PFS times of less than 6 months had the highest pre-treatment levels of TKa and little to no on-treatment increase of TKa. Patients who had a PFS time of between 6-24 months, had a low pre-treatment TKa but a less than 2-fold on-treatment increase of TKa. Patients who had a response to treatment lasting longer than 24 months, had a low pre-treatment TKa level and a 2-fold or greater on-treatment increase of TKa. This is indicative of a proliferative burst of T-cells in patients within the first month of successful ICI treatment.

## Discussion

In the patients with advanced cutaneous melanoma included in the study, significantly higher TKa levels were seen in patients that at treatment start had poor performance status, more advanced tumour stage, and higher LDH level. The median TKa was 42 Du/L (range <20-1787 Du/L), while, as a reference, in 123 healthy subjects, the median TKa value was <20 Du/L (data not shown). In a cohort of preoperative pancreatic cancer patients, the median TKa value was 40 Du/L, and in a cohort of preoperative renal cell cancer patients, the median TKa was 38 Du/L [12,14]. Further, in a cohort of non-small cell lung cancer patients, the median TKa was 129 Du/L before the start of systemic treatment, whereas in a cohort of breast cancer patients, the pre-treatment TKa was 57 Du/L in patients with locoregional disease and 101 Du/L in patients with visceral metastasis [15,18]. Collectively, this data show that TKa levels in patients with metastatic melanoma is, as for the other studied cancer types, elevated compared to the levels in healthy individuals and higher in patients with more advanced disease.

The patients with high TKa had a significantly poorer response to the ICI treatment and also a significantly shorter survival (both PFS and OS). In the multivariate analysis, TKa was not an independent predictor for PFS and OS. The bivariate analysis showed that TKa association with PFS and OS was, in a varying degree, dependent on ECOG, LDH, and tumour stage; however, a considerable fraction of patients did not have corresponding pairs of good or poor baseline variables (Figure 5). Clinical factors such as performance status, tumour stage, and tumour burden are well-known prognostic factors and are also predictive of ICI efficacy in melanoma [1-4,16]. As for serum markers, elevated LDH level is the strongest known prognostic and predictive factor and is the only biomarker that is routinely used when monitoring melanoma patients in the clinic and included as a marker in clinical trials [16]. The serum LDH level reflects the hypoxic environment often present in melanoma tumours, with reduced oxidative phosphorylation and increased anaerobic glycolysis, where LDH catalyses the conversion of pyruvate to lactate when oxygen supply is low or absent [19]. LDH is not a secreted enzyme; thus, an elevated serum level is thought to be secondary to spillage of LDH when melanoma cells outgrow their blood supply. LDH is also often elevated in various conditions affecting the liver, both malignant and non-malignant [19]. The TK enzyme has a significant role in DNA synthesis and repair. These processes are highly active in proliferating cells and dividing tumour cells release TK during mitotic exit [7,8]. Hence, LDH and TKa are both markers of cell proliferation and tumour burden, but through different cellular processes.

The data herein also shows that a positive patient response to an ICI can be predicted by measuring TKa levels in cancer patients at 2 key timepoints - before treatment and 1-4 weeks after the first ICI treatment dose. If the before treatment TKa level is low, this indicates a level of disease burden that can effectively be managed by the immune system once it is activated. If the ICI on-treatment TKa level increases by at least 2-fold as compared to the baseline level, that indicates a successful response to the therapy and a sufficient increase in T-cell activation and proliferation to achieve tumour killing.

Several other markers have been reported as predicative for ICI efficacy, including the composition of peripheral blood leukocytes, circulating tumour DNA(ctDNA) and exosomes, tumour mutational burden (TMB), high interferon-gamma-related gene expression signature in tumours, diversity of the gut microbiome, and invasive tumor-biopsy tests (currently used for patient selection) measuring the expression of ICI-receptors [20-27]. In the clinical setting, widespread implementation of predictive assays, such as TMB, ctDNA, exosomes, tumour RNA expression signatures, or microbiome analyses, is a challenge, e.g. due to the complex and costly techniques and equipment needed, and there are also many different assays that can be used. To measure TKa is a simpler and less costly test (being ELISA based) for a single plasma marker, and the assay can readily be set up in regular hospital laboratories.

## Conclusions

High pre-treatment plasma TKa levels were significantly associated with worse baseline characteristics and poor response and survival in ICI-treated melanoma patients. The inventors have identified for the first time that TKa is an interesting, previously not explored, biomarker in cancer patients that can be used to indicate response to subsequent ICI treatment.

## Tables

**Table 1:** Pre-treatment thymidine kinase 1 (TK) activity in (Du/L) in plasma patients with unresectable melanoma.

| Characteristics | TK activity (Du/L) median (range) | *P* value |
|---|---|---|
| Sex | | |
|     Male (n=60) | 37 (<20-1787) | |

(continued)

| Characteristics | TK activity (Du/L) median (range) | P value |
|---|---|---|
| Female (n=30) | 53 (<20-869) | 0. 689 |
| Age | | |
| ≤65 years (n=40) | 37 (<20-1111) | |
| >65 years (n=50) | 55 (<20-1787) | 0.111 |
| BRAF v600 mutation in tumor | | |
| Yes (n=38) | 46 (<20-1649) | 0.649 |
| No (n=52) | 40 (<20-1787) | |
| Performance status | | |
| ECOG 0 (n=70) | 35 (<20-1787) | |
| ECOG ≥1 (n=20) | 138 (<20-1650) | 0.003 |
| LDH | | |
| Normal LDH (n=44) | 34 (<20-242) | |
| Elevated LDH (n=46) | 71 (<20-1787) | <0.001 |
| Tumor stage | | |
| M1a or M1b (n=48) | 35 (<20-1649) | |
| M1c or M1d (n=42) | 66 (<20-1787) | 0.015 |
| Numbers of affected organs | | |
| 1-2 affected organs (n=58) | 36 (<20-1649) | |
| ≥3 affected organs (n=32) | 64 (<20-1787) | 0.066 |
| Affected organs (patients in stage M1a) | | |
| Soft tissue (n=29) | 34 (<20-1649) | |
| Affected organs (patients in stage M1b-M1d) | | |
| Soft tissue | | |
| Yes (n=46) | 47 (<20-1787) | |
| No (n=15) | 59 (<20-708) | 0.653 |
| Lung | | |
| Yes (n=42) | 49 (<20-1650) | |
| No (n=19) | 67 (<20-1787) | 0.818 |
| Liver | | |
| Yes (n=18) | 58 (<20-1787) | |
| No (n=43) | 44 (<20-1195) | 0.195 |
| Bone | | |
| Yes (n=15) | 67 (<20-1111) | |
| No (n=46) | 42 (<20-1787) | 0.886 |
| Brain | | |
| Yes (n=13) | 77 (<20-1111) | |
| No (n=48) | 51 (<20-1787) | 0.280 |
| Other | | |
| Yes (n=19) | 70 (<20-1787) | |
| No (n=42) | 39 (<20-1650) | 0.137 |
| Previous lines of treatment, n (%) | | |
| 0 previous lines (n=77) | 37 (<20-1787) | 0.261 |
| ≥1 previous lines (n=13) | 77 (34-1650) | |

**Table 2:** Characteristics of melanoma patients with low (<60 Du/L) or high thymidine kinase 1 (TK) activity (Du/L) in plasma before starting immune checkpoint inhibitor treatment.

| | TKa low | TKa high | P value |
|---|---|---|---|
| Patients, n (%) | 57 (63.3%) | 33 (36.7%) | |
| TK (Du/L), median (range) | 39 (<20-59) | 140 (62-1787) | <0.001 |

(continued)

|  | TKa low | TKa high | P value |
|---|---|---|---|
| Sex, n (%) |  |  |  |
| Male | 42 (73.7%) | 17 (58.6%) | 0.155 |
| Female | 15 (26.3%) | 12 (41.4%) |  |
| Age, median (range) |  |  |  |
| Age, years | 64 (31-84) | 71 (34-84) | 0.065 |
| Performance status, n (%) |  |  |  |
| ECOG 0 | 52 (91.2%) | 17 (58.6%) | <0.001 |
| ECOG ≥1 | 5 (8.8%) | 12 (41.4%) |  |
| BRAF mutation in tumor, n (%) |  |  |  |
| Yes | 33 (57.9%) | 19 (65.5%) | 0.494 |
| No | 24 (42.1%) | 10 (34.5%) |  |
| Tumor stage, n (%) |  |  |  |
| M1a or M1b | 38 (66.7%) | 9 (31.0%) | 0.002 |
| M1c or M1d | 19 (33.3%) | 20 (69.0%) |  |
| Affected organs, n (%) |  |  |  |
| 1-2 affected organs | 41 (71.9%) | 14 (48.3%) | 0.031 |
| ≥3 affected organs | 16 (28.1%) | 15 (51.7%) |  |
| LDH, median (range) |  |  |  |
| LDH, μkat/L | 3.6 (1.7-14.2) | 4.9 (3.2-37.0) | <0.001 |
| LDH, n (%) |  |  |  |
| Normal LDH | 36 (63.2%) | 7 (24.1%) | <0.001 |
| Elevated LDH | 21 (36.8%) | 22 (75.9%) |  |
| Previous lines of treatment, n (%) |  |  |  |
| 0 previous lines | 52 (91.2%) | 25 (86.2%) | 0.517 |
| ≥1 previous lines | 5 (8.8%) | 4 (13.8%) |  |
| ICI regime* |  |  |  |
| CTLA-4 inhibitor single | 1 (1.8%) | 1 (3.4%) | 0.182 |
| PD-1 inhibitor single | 50 (88%) | 28 (97%) |  |
| CTLA-4 and PD-1 inhibitor | 6 (10%) | 0 (0%) |  |

*Immune checkpoint inhibitor regime that the patient started after the baseline TKa test

**Table 3:** Response evaluations in melanoma patients with low (<60 Du/L) or high thymidine kinase 1 (TK) activity (Du/L) in plasma before starting immune checkpoint inhibitor treatment.

|  | TKa low | TKa high | P value |
|---|---|---|---|
| Best overall response, n (%) |  |  |  |
| Complete response | 19 (33.3%) | 2 (6.0%) |  |
| Partial response (PR) | 17 (29.8.1%) | 10 (30.3%) |  |
| Stable disease (SD) | 10 (17.5%) | 6 (18.2%) |  |
| Progressive disease (PD) | 11 (19.3%) | 15 (45.5%) |  |
| Complete response rate (CR), % | 33.3% | 6.0% | 0.016 |
| Response rate (CR+PR), % | 63.2% | 30.3% | 0.022 |
| Disease control rate (CR+PR+SD), % | 80.7% | 54.5% | 0.022 |
| Treatment stopped due to |  |  |  |
| Progressive disesae | 24 (42.1%) | 17 (51.5%) | 0.543 |
| Adequate response | 22 (38.6%) | 9 (27.3%) |  |
| Toxicity | 11 (19.2%) | 7 (21.2%) |  |

**Table 4:** Cox regressions for survival in metastatic melanoma patients treated with immune checkpoint inhibitors.

| | Univariate analyses | | | Multivariate analyses | | |
|---|---|---|---|---|---|---|
| | HR | 95% CI | *P value* | HR | 95% CI | *P value* |
| **Progression free survival** | | | | | | |
| Age (old vs. young) | 0.89 | (0.51- 1.54) | 0.6769 | 0.79 | (0.44 - 1.43) | 0.4435 |
| Sex (male vs. female) | 1.71 | (0.99 - 2.96) | 0.0523 | 1.56 | (0.84 - 2.87) | 0.1573 |
| ECOG (≥1 vs. 0) | 2.62 | (1.44 - 4.74) | 0.0015 | 2.04 | (0.93 - 4.45) | 0.0745 |
| Tumor stage (M1a-b vs. M1c-d) | 2.01 | (1.17 - 3.46) | 0.0117 | 1.63 | (0.89 - 2.98) | 0.1164 |
| LDH (elevated vs. normal) | 1.88 | (1.12 - 3.15) | 0.0170 | 1.99 | (1.12 - 3.55) | 0.0192 |
| TK activity (high vs. low) | 1.83 | (1.08 - 3.08) | 0.0238 | 0.78 | (0.39 - 1.56) | 0.4842 |
| **Overall survival** | | | | | | |
| Age (old vs. young) | 1.22 | (0.67 - 2.21) | 0.5174 | 1.14 | (0.60 - 2.17) | 0.6883 |
| Sex (male vs. female) | 2.52 | (1.31 - 4.85) | 0.0055 | 2.35 | (1.13 - 4.85) | 0.0214 |
| ECOG (≥1 vs. 0) | 3.42 | (1.77 - 6.60) | 0.0002 | 2.07 | (0.87 - 4.91) | 0.0993 |
| Tumor stage (M1a-b vs. M1c-d) | 2.99 | (1.55 - 5.73) | 0.0010 | 2.22 | (1.09 - 4.54) | 0.0288 |
| LDH (elevated vs. normal) | 2.29 | (1.26 - 4.15) | 0.0063 | 2.15 | (1.12 - 4.14) | 0.0213 |
| TK activity (high vs. low) | 2.25 | (1.25 - 4.05) | 0.0067 | 0.82 | (0.39 - 1.75) | 0.6129 |

**Table 5.** TK activity pre-treatment (pre-trm), on-treatment (on-trm) and at end of treatment (eot) with immunotherapy.

| | **TKa PRE-TRM** | **TKa ON-TRM** | **TKa EOT** |
|---|---|---|---|
| **PFS<6months** | 54 | 58 | 148 |
| **PFS>6months** | 36 | 44 | 147 |
| **PFS>24 months** | 34 | 99 | 44 |

## EXAMPLE 2

A phase 1b study of the CDK4/6 inhibitor ribociclib in combination with the PD-1 inhibitor spartalizumab (aka PDR001) was carried out in patients with hormone receptor-positive metastatic breast cancer (HR+ MBC) and metastatic ovarian cancer (MOC).

The study enrolled 24 patients. Ribociclib was taken orally once a day on days 1-21 of a 28-day cycle. Spartalizumab was given intravenously on day 1 of the same 28-day cycle. Blood was drawn from each patient at baseline (pre-treatment) and on day 1 of every 28 day cycle of therapy. Plasma was isolated from the blood samples which were then analyzed for thymidine kinase activity using the DiviTum-TKa assay as previously described. The data is represented in Figure 7 and in the accompanying data table, Table 6.

As was seen in the melanoma PD-1 inhibitor study with DiviTim-TKa, patients with the best clinical responses had both a low baseline TKa value and a 2-fold or greater on-treatment increase in TKa.

Patient #19 was a breast cancer patient that had a baseline TKa level of 590 DuA. After the first cycle of therapy, on day 28 the patient's TKa level increased 2-fold to 1173 DuA. This patient had a partial response to therapy with a tumor volume decrease of 68% and the patient remained on therapy for 334 days. This was the 2nd longest duration on therapy. Patient number 27 was also a breast cancer patient. This patient's baseline TKa level was 174 DuA (the lowest TKa baseline level in the study). After the first cycle of therapy, on day 28 the patient's TKa level increased 3.8 fold to 411 DuA (the highest fold increase on the study). This patient had a partial response to therapy with a tumor volume decrease of 54% and the patient remained on therapy for 411 days. This was the longest duration on therapy in this study. None of the other 22 patients in the study (besides #19 and #27 as described above) met the criteria of BOTH a low baseline level of TKa and a two-fold on-therapy increase in TKa. And subsequently none derived any meaningful clinical benefit. The only exception was patient #3. This patient had a low BL level of TKa (296 DuA) along with a 2.6 fold TKa increase on therapy to 758 DuA but this patient only remained on therapy for 95 days. Upon further investigation, it was discovered that this patient was experiencing clinical symptoms and that was the reason for the treatment discontinuation. Had this patient been able to remain on therapy, it is entirely plausible that the patient would have derived benefit. Patient #24 also achieved a greater than 2 fold on-therapy TKa increase (2.21) however the pateint's baseline TKa was extremely high (1316 DuA) so the patient did not meet the criteria for predicting immune checkpoint inhibitor benefit, based on a pre-treatment TKa cut-off value of 550 DuA +/- 20%, and indeed had rapid progressive disease in 35 days.

This data adds to and builds on the previous data with TKa exemplified in melanoma patients treated with immune checkpoint inhibitors (ICIs). This data confirms the applicability of the methods of the invention for other cancers, such as the exemplified metastatic HR+ breast cancer and ovarian cancer. It also confirms applicability of the method when using a different ICI (spartalizumab).

**Table 6**. Data Table for Figure 7.

| PATIENT # | BASELINE TKa in DuA | Cycle 2 Day 1 TKa in DuA | FOLD change | RESPONSE | TUMOR TYPE |
|---|---|---|---|---|---|
| 2 | 1879 | 2215 | 1.18 | PD | BREAST |
| 3 | 296 | 758 | 2.56 | SD<6M | BREAST |
| 4 | 1472 | 1374 | 0.93 | PD | BREAST |
| 5 | 922 | 774 | 0.84 | PD | BREAST |
| 6 | 7284 | 7284 | 1.51 | NE | BREAST |
| 8 | 998 | 1057 | 1.06 | PD | BREAST |
| 11 | 1143 | 1597 | 1.40 | SD<6M | BREAST |
| 12 | 381 | 393 | 1.03 | SD>=6M | BREAST |
| 13 | 1127 | 1219 | 1.08 | SD<6M | BREAST |
| 14 | 1273 | 918 | 0.72 | SD<6M | BREAST |
| 15 | 1463 | 602 | 0.41 | SD>=6M | BREAST |
| 16 | 5637 | 3587 | 0.64 | SD<6M | BREAST |
| 18 | 673 | 727 | 1.08 | PD | OVARIAN |
| 19 | 590 | 1173 | 1.99 | PR | BREAST |
| 20 | 607 | 615 | 1.01 | SD<6M | OVARIAN |
| 22 | 168 | 308 | 1.83 | SD<6M | OVARIAN |
| 24 | 1316 | 2913 | 2.21 | PD | OVARIAN |
| 25 | 895 | 1111 | 1.24 | SD<6M | OVARIAN |
| 26 | 1622 | 839 | 0.52 | PD | BREAST |
| 27 | 174 | 663 | 3.81 | PR | BREAST |
| 28 | 1305 | 1113 | 0.85 | PD | BREAST |
| 29 | 1715 | 1773 | 1.03 | PD | BREAST |
| 31 | 692 | 836 | 1.21 | PD | BREAST |
| 32 | 1199 | 840 | 0.70 | SD<6M | BREAST |

### References

[1] Hodi FS, O'Day SJ, McDermott DF, Weber RW, Sosman JA, Haanen JB, et al. Improved survival with ipilimumab in patients with metastatic melanoma. N Engl J Med. 2010;363:711-23.

[2] Robert C, Long GV, Brady B, Dutriaux C, Maio M, Mortier L, et al. Nivolumab in previously untreated melanoma without BRAF mutation. N Engl J Med. 2015;372:320-30.

[3] Robert C, Schachter J, Long GV, Arance A, Grob JJ, Mortier L, et al. Pembrolizumab versus Ipilimumab in Advanced Melanoma. N Engl J Med. 2015;372:2521-32.

[4] Larkin J, Chiarion-Sileni V, Gonzalez R, Grob JJ, Cowey CL, Lao CD, et al. Combined Nivolumab and Ipilimumab or Monotherapy in Untreated Melanoma. N Engl J Med. 2015;373:23-34.

[5] Robert C, Karaszewska B, Schachter J, Rutkowski P, Mackiewicz A, Stroiakovski D, et al. Improved overall survival in melanoma with combined dabrafenib and trametinib. N Engl J Med. 2015;372:30-9.

[6] Dummer R, Ascierto PA, Gogas HJ, Arance A, Mandala M, Liszkay G, et al. Encorafenib plus binimetinib versus vemurafenib or encorafenib in patients with BRAF-mutant melanoma (COLUMBUS): a multicentre, open-label, randomised phase 3 trial. Lancet Oncol. 2018;19:603-15.

[7] Schwartz JL, Tamura Y, Jordan R, Grierson JR, Krohn KA. Monitoring tumor cell proliferation by targeting DNA synthetic processes with thymidine and thymidine analogs. J Nucl Med. 2003;44:2027-32.

[8] Bitter EE, Townsend MH, Erickson R, Allen C, O'Neill KL. Thymidine kinase 1 through the ages: a comprehensive review. Cell Biosci. 2020;10:138.

[9] Bagegni N, Thomas S, Liu N, Luo J, Hoog J, Northfelt DW, et al. Serum thymidine kinase 1 activity as a pharmacodynamic marker of cyclin-dependent kinase 4/6 inhibition in patients with early-stage breast cancer receiving neoadjuvant palbociclib. Breast Cancer Res. 2017;19:123.

[10] McCartney A, Bonechi M, De Luca F, Biagioni C, Curigliano G, Moretti E, et al. Plasma Thymidine Kinase Activity as a Biomarker in Patients with Luminal Metastatic Breast Cancer Treated with Palbociclib within the TREnd Trial. Clin Cancer Res. 2020;26:2131-9.

[11] Cabel L, Rosenblum D, Lerebours F, Brain E, Loirat D, Bergqvist M, et al. Plasma thymidine kinase 1 activity and outcome of ER+ HER2- metastatic breast cancer patients treated with palbociclib and endocrine therapy. Breast Cancer Res. 2020;22:98.

[12] Felix K, Hinz U, Dobiasch S, Hackert T, Bergmann F, Neumuller M, et al. Preoperative Serum Thymidine Kinase Activity as Novel Monitoring, Prognostic, and Predictive Biomarker in Pancreatic Cancer. Pancreas. 2018;47:72-9.

[13] McCartney A, Biagioni C, Schiavon G, Bergqvist M, Mattsson K, Migliaccio I, et al. Prognostic role of serum thymidine kinase 1 activity in patients with hormone receptor-positive metastatic breast cancer: Analysis of the randomised phase III Evaluation of Faslodex versus Exemestane Clinical Trial (EFECT). Eur J Cancer. 2019;114:55-66.

[14] Nisman B, Appelbaum L, Yutkin V, Nechushtan H, Hubert A, Uziely B, et al. Serum Thymidine Kinase 1 Activity Following Nephrectomy for Renal Cell Carcinoma and Radiofrequency Ablation of Metastases to Lung and Liver. Anticancer Res. 2016;36:1791-7.

[15] Nisman B, Nechushtan H, Biran H, Gantz-Sorotsky H, Peled N, Gronowitz S, et al. Serum thymidine kinase 1 activity in the prognosis and monitoring of chemotherapy in lung cancer patients: a brief report. J Thorac Oncol. 2014;9:1568-72.

[16] Gershenwald JE, Scolyer RA, Hess KR, Sondak VK, Long GV, Ross MI, et al. Melanoma staging: Evidence-based changes in the American Joint Committee on Cancer eighth edition cancer staging manual. CA Cancer J Clin. 2017;67:472-92.

[17] Eisenhauer EA, Therasse P, Bogaerts J, Schwartz LH, Sargent D, Ford R, et al. New response evaluation criteria in solid tumours: revised RECIST guideline (version 1.1). Eur J Cancer. 2009;45:228-47.

[18] Bjohle J, Bergqvist J, Gronowitz JS, Johansson H, Carlsson L, Einbeigi Z, et al. Serum thymidine kinase activity compared with CA 15-3 in locally advanced and metastatic breast cancer within a randomized trial. Breast Cancer Res Treat. 2013;139:751-8.

[19] Palmer SR, Erickson LA, Ichetovkin I, Knauer DJ, Markovic SN. Circulating serologic and molecular biomarkers in malignant melanoma. Mayo Clin Proc. 2011;86:981-90.

[20] Fujii T, Naing A, Rolfo C, Hajjar J. Biomarkers of response to immune checkpoint blockade in cancer treatment. Crit Rev Oncol Hematol. 2018;130:108-20.

[21] Pico de Coana Y, Wolodarski M, van der Haar Avila I, Nakajima T, Rentouli S, Lundqvist A, et al. PD-1 checkpoint blockade in advanced melanoma patients: NK cells, monocytic subsets and host PD-L1 expression as predictive biomarker candidates. Oncoimmunology. 2020;9:1786888.

[22] Hugo W, Zaretsky JM, Sun L, Song C, Moreno BH, Hu-Lieskovan S, et al. Genomic and Transcriptomic Features of Response to Anti-PD-1 Therapy in Metastatic Melanoma. Cell. 2016;165:35-44.

[23] Snyder A, Makarov V, Merghoub T, Yuan J, Zaretsky JM, Desrichard A, et al. Genetic basis for clinical response to CTLA-4 blockade in melanoma. N Engl J Med. 2014;371:2189-99.

[24] Rozeman EA, Hoefsmit EP, Reijers ILM, Saw RPM, Versluis JM, Krijgsman O, et al. Survival and biomarker analyses from the OpACIN-neo and OpACIN neoadjuvant immunotherapy trials in stage III melanoma. Nat Med. 2021;27:256-63.

[25] Grasso CS, Tsoi J, Onyshchenko M, Abril-Rodriguez G, Ross-Macdonald P, Wind-Rotolo M, et al. Conserved Interferon-gamma Signaling Drives Clinical Response to Immune Checkpoint Blockade Therapy in Melanoma. Cancer Cell. 2020;38:500-15 e3.

[26] Kaminska P, Buszka K, Zabel M, Nowicki M, Alix-Panabieres C, Budna-Tukan J. Liquid Biopsy in Melanoma: Significance in Diagnostics, Prediction and Treatment Monitoring. Int J Mol Sci. 2021;22.

[27] Gopalakrishnan V, Spencer CN, Nezi L, Reuben A, Andrews MC, Karpinets TV, et al. Gut microbiome modulates response to anti-PD-1 immunotherapy in melanoma patients. Science. 2018;359:97-103.

[28] Kim KH, Cho J, Ku BM, Koh J, Sun JM, Lee SH, Ahn JS, Cheon J, Min YJ, Park SH, Park K, Ahn MJ, Shin EC. The First-week Proliferative Response of Peripheral Blood PD-1+CD8+ T Cells Predicts the Response to Anti-PD-1 Therapy in Solid Tumors. Clin Cancer Res. 2019 Apr 1;25(7):2144-2154.

## Claims

1. A method for determining the prognosis of an individual having cancer and currently being treated with one or more immune checkpoint inhibitors, the method comprising or consisting of the steps of:

   a) providing a sample obtained from the individual prior to treatment with one or more immune checkpoint inhibitors;
   b) measuring the activity and/or concentration of thymidine kinase (TK) in the sample;
   c) providing a sample obtained from the individual 1-4 weeks after first treatment with the one or more immune checkpoint inhibitors; and
   d) measuring the activity and/or concentration of thymidine kinase (TK) in the sample provided in step (c);

   wherein the activity and/or concentration of thymidine kinase (TK) measured in steps (b) and (d) is indicative of the prognosis of the individual if further treated with one or more immune checkpoint inhibitors, wherein the prognosis of the individual if further treated with one or more checkpoint inhibitors is positive when the activity and/or concentration of thymidine kinase (TK) measured in step (d) is increased compared to the activity and/or concentration of thymidine kinase (TK) measured in step (b).

2. The method of claim 1 wherein the method is for stratifying a patient or patients having cancer for further treatment with one or more immune checkpoint inhibitors.

3. A method for stratifying a patient having cancer for further treatment with one or more immune checkpoint inhibitors, the method comprising or consisting of the steps of:

   i) performing the method of any previous claim; and
   ii) stratifying the patient for further treatment with one or more immune checkpoint inhibitors or an alternative cancer therapy based on the outcome of step (i), wherein the patient is stratified for further treatment with one or more immune checkpoint inhibitors when the activity and/or concentration of thymidine kinase (TK) measured in step (d) is increased compared to the activity and/or concentration of thymidine kinase (TK) measured in step (b).

4. The method of any previous claim wherein the prognosis of the individual if further treated with one or more checkpoint inhibitors is positive (for example the individual will have progression free survival of more than 24 months) and/or the patient is stratified for further treatment with one or more immune checkpoint inhibitors when the activity and/or concentration of thymidine kinase (TK) measured in step (d) is increased by at least 100% compared to the activity and/or concentration of thymidine kinase (TK) measured in step (b).

5. The method of any previous claim wherein the prognosis of the individual if further treated with one or more immune checkpoint inhibitors is positive and/or the patient is stratified for further treatment with one or more immune checkpoint inhibitors when the activity and/or concentration of thymidine kinase (TK) measured in step (b) is lower than a cut off value.

6. The method of any previous claim wherein the prognosis of the individual if further treated with one or more immune checkpoint inhibitors is negative (for example the individual would have progression free survival of less than 6 months) and/or the patient is stratified for no further treatment or treatment with an alternative cancer therapy to one or more immune checkpoint inhibitors when the activity and/or concentration of thymidine kinase (TK) measured in step (b) is greater than or equal to a cut off value.

7. The method of any previous claim wherein the prognosis of the individual if further treated with one or more immune checkpoint inhibitors is positive (for example the individual will have progression free survival of at least 6 months) and/or the patient is stratified for further treatment with one or more immune checkpoint inhibitors when the activity and/or concentration of thymidine kinase (TK) measured in step (b) is lower than a cut off value, and wherein the activity and/or concentration of thymidine kinase (TK) measured in step (d) is increased compared to the activity and/or concentration of thymidine kinase (TK) measured in step (b); optionally wherein the prognosis of the individual if further treated with one or more immune checkpoint inhibitors is positive (for example the individual will have progression free survival of more than 24 months) and/or the patient is stratified for further treatment with one or more immune checkpoint inhibitors when the activity and/or concentration of thymidine kinase (TK) measured in step (b) is lower than a cut off value, and wherein the activity and/or concentration of thymidine kinase (TK) measured in step (d) is increased by at least 100% compared to the activity and/or concentration of thymidine kinase (TK) measured in step (b).

8. The method of any previous claim wherein the prognosis of the individual if further treated with one or more immune checkpoint inhibitors is negative (for example the individual would have progression free survival of less than 6 months) and/or the patient is stratified for no further treatment or treatment with an alternative cancer therapy to one or more immune checkpoint inhibitors when the activity and/or concentration of thymidine kinase (TK) measured in step (b) is greater than or equal to a cut off value, and wherein the activity and/or concentration of thymidine kinase (TK) measured in step (d) is not increased compared to the activity and/or concentration of thymidine kinase (TK) measured in step (b).

9. The method of any one of claims 6-8 wherein the activity of thymidine kinase (TKa) is measured in step (b) and the cut off value is 550 DuA +/- 20%.

10. The method of any previous claim wherein the method further comprises the steps of:

    (e) providing one or more control samples; and
    (f) measuring the activity and/or concentration of thymidine kinase (TK) in the sample provided in step (e);

wherein the prognosis of the individual if further treated with one or more immune checkpoint inhibitors is determined by comparing the measurement in step (f) with the measurement in step (b) and/or step (d); optionally wherein the one or more control sample was obtained from an individual having cancer before subsequently undergoing successful treatment with one or more immune checkpoint inhibitors.

11. The method of claim 10 wherein the prognosis of the individual if further treated with one or more immune checkpoint inhibitors is positive and/or the patient is stratified for further treatment with one or more immune checkpoint inhibitors when the activity and/or concentration of thymidine kinase (TK) measured in step (b) corresponds to or is lower than the activity of thymidine kinase (TKa) measured in step (f), optionally

    wherein the one or more control sample was obtained from an individual having cancer before subsequently undergoing unsuccessful treatment with one or more immune checkpoint inhibitors; and/or
    wherein the prognosis of the individual if further treated with one or more immune checkpoint inhibitors is negative and/or the patient is stratified for no further treatment or treatment with an alternative cancer therapy to one or more immune checkpoint inhibitors when the activity and/or concentration of thymidine kinase (TK) measured in step (b) is higher than the activity and/or concentration of thymidine kinase (TK) measured in step (f).

12. The method of any previous claim further comprising the step of:

    g) measuring the activity and/or concentration of lactate dehydrogenase (LDH) in the sample provided in step (a); wherein the activity and/or concentration of LDH measured in step (g) is additionally indicative of the prognosis of the individual if subsequently treated with one or more immune checkpoint inhibitors; optionally wherein the prognosis of the individual if treated or further treated with one or more immune checkpoint inhibitors is positive (for example the individual will have progression free survival of more than 24 months) and/or the patient is stratified for treatment or further treatment with one or more immune checkpoint inhibitors when the activity and/or concentration of LDH measured in step (g) is lower than a cut off value; optionally wherein the activity of LDH is measured and the cut off value is selected from between 2 and 6 mikrokat/L, for example between 3 and 5 mikrokat/L, or for example is 4 mikrokat/L.

13. One or more immune checkpoint inhibitors for use in treating cancer in a patient wherein the patient has been selected for further treatment with one or more immune checkpoint inhibitors using a method for stratifying a patient according to any one of claims 2-12,
wherein the patient is selected for further treatment due to having a serum or plasma TK activity and/or concentration 1-4 weeks after treatment with an ICI inhibitor which is increased compared to their serum or plasma TK activity and/or concentration prior to ICI treatment; optionally wherein the individual is selected for further treatment due to also having a serum or plasma TK activity and/or concentration prior to ICI treatment which is lower than a cut off value.

14. The method or immune checkpoint inhibitor for use of any previous claim wherein:

   (i) it is the activity of thymidine kinase (TKa) which is measured;
   (ii) the cancer is a cancer approved for treatment with an immune checkpoint inhibitor;
   (iii) the cancer is selected from the group comprising: melanoma; breast cancer; and/or ovarian cancer;
   (iv) the one or more immune checkpoint inhibitors are selected from the list consisting of: CTLA-4 inhibitor, PD-1 inhibitor, PD-L1 inhibitor, LAG-3 inhibitor;
   (v) the individual or patient is a human; and/or
   (vi) the sample is a plasma or serum sample.

15. Use of thymidine kinase: (i) as a prognostic biomarker for individuals having cancer being currently treated with immune checkpoint inhibitors; or (ii) for stratifying patients having cancer for further treatment with immune checkpoint inhibitors; wherein the prognosis of the individual if further treated with one or more checkpoint inhibitors is positive and/or wherein the patient is stratified for further treatment with one or more immune checkpoint inhibitors when the activity and/or concentration of thymidine kinase (TK) measured in step (d) is increased compared to the activity and/or concentration of thymidine kinase (TK) measured in step (b).

**Patentansprüche**

1. Verfahren zum Bestimmen der Prognose einer Person, die Krebs hat und derzeit mit einem oder mehreren Immun-Checkpoint-Inhibitoren behandelt wird, wobei das Verfahren die folgenden Schritte umfasst oder aus diesen besteht:

   a) Bereitstellen einer Probe, die von der Person vor der Behandlung mit einem oder mehreren Immun-Checkpoint-Inhibitoren erhalten wurde;
   b) Messen der Aktivität und/oder Konzentration der Thymidinkinase (TK) in der Probe;
   c) Bereitstellen einer Probe, die von der Person 1-4 Wochen nach der ersten Behandlung mit dem einen oder den mehreren Immun-Checkpoint-Inhibitoren erhalten wurde; und
   d) Messen der Aktivität und/oder Konzentration der Thymidinkinase (TK) in der in Schritt (c) bereitgestellten Probe;

   wobei die in den Schritten (b) und (d) gemessene Aktivität und/oder Konzentration der Thymidinkinase (TK) auf die Prognose der Person hindeutet, wenn diese mit einem oder mehreren Immun-Checkpoint-Inhibitoren weiterbehandelt wird, wobei die Prognose der Person, wenn diese mit einem oder mehreren Checkpoint-Inhibitoren weiterbehandelt wird, positiv ist, wenn die in Schritt (d) gemessene Aktivität und/oder Konzentration der Thymidinkinase (TK) im Vergleich zu der in Schritt (b) gemessenen Aktivität und/oder Konzentration der Thymidinkinase (TK) erhöht ist.

2. Verfahren nach Anspruch 1, wobei das Verfahren zum Stratifizieren eines Patienten oder von Patienten mit Krebs zur Weiterbehandlung mit einem oder mehreren Immun-Checkpoint-Inhibitoren dient.

3. Verfahren zum Stratifizieren eines Patienten mit Krebs zur Weiterbehandlung mit einem oder mehreren Immun-Checkpoint-Inhibitoren, wobei das Verfahren die folgenden Schritte umfasst oder aus diesen besteht:

   i) Durchführen des Verfahrens nach irgendeinem der vorhergehenden Ansprüche; und
   ii) Stratifizieren des Patienten zur Weiterbehandlung mit einem oder mehreren Immun-Checkpoint-Inhibitoren oder einer alternativen Krebstherapie basierend auf dem Ausgang von Schritt (i), wobei der Patient zur Weiterbehandlung mit einem oder mehreren Immun-Checkpoint-Inhibitoren stratifiziert wird, wenn die in Schritt (d) gemessene Aktivität und/oder Konzentration der Thymidinkinase (TK) im Vergleich zu der in Schritt (b) gemessenen Aktivität und/oder Konzentration der Thymidinkinase (TK) erhöht ist.

4. Verfahren nach irgendeinem der vorhergehenden Ansprüche, wobei die Prognose der Person, wenn diese mit einem oder mehreren Checkpoint-Inhibitoren weiterbehandelt wird, positiv ist (zum Beispiel wird die Person ein progressionsfreies Überleben von mehr als 24 Monaten haben) und/oder der Patient zur Weiterbehandlung mit einem oder mehreren Immun-Checkpoint-Inhibitoren stratifiziert wird, wenn die in Schritt (d) gemessene Aktivität und/oder Konzentration der Thymidinkinase (TK) im Vergleich zu der in Schritt (b) gemessenen Aktivität und/oder Konzentration der Thymidinkinase (TK) um mindestens 100 % erhöht ist.

5. Verfahren nach irgendeinem der vorhergehenden Ansprüche, wobei die Prognose der Person, wenn diese mit einem oder mehreren Immun-Checkpoint-Inhibitoren weiterbehandelt wird, positiv ist und/oder der Patient zur Weiterbehandlung mit einem oder mehreren Immun-Checkpoint-Inhibitoren stratifiziert wird, wenn die in Schritt (b) gemessene Aktivität und/oder Konzentration der Thymidinkinase (TK) niedriger als ein Cut-off-Wert ist.

6. Verfahren nach irgendeinem der vorhergehenden Ansprüche, wobei die Prognose der Person, wenn diese mit einem oder mehreren Immun-Checkpoint-Inhibitoren weiterbehandelt wird, negativ ist (zum Beispiel würde die Person ein progressionsfreies Überleben von weniger als 6 Monaten haben) und/oder der Patient für keine Weiterbehandlung oder Behandlung mit einer alternativen Krebstherapie zu einem oder mehreren Immun-Checkpoint-Inhibitoren stratifiziert wird, wenn die in Schritt (b) gemessene Aktivität und/oder Konzentration der Thymidinkinase (TK) größer als oder gleich einem Cut-off-Wert ist.

7. Verfahren nach irgendeinem der vorhergehenden Ansprüche, wobei die Prognose der Person, wenn diese mit einem oder mehreren Immun-Checkpoint-Inhibitoren weiterbehandelt wird, positiv ist (zum Beispiel wird die Person ein progressionsfreies Überleben von mindestens 6 Monaten haben) und/oder der Patient zur Weiterbehandlung mit einem oder mehreren Immun-Checkpoint-Inhibitoren stratifiziert wird, wenn die in Schritt (b) gemessene Aktivität und/oder Konzentration der Thymidinkinase (TK) niedriger als ein Cut-off-Wert ist, und wobei die Aktivität und/oder Konzentration der in Schritt (d) gemessenen Thymidinkinase (TK) im Vergleich zu der in Schritt (b) gemessenen Aktivität und/oder Konzentration der Thymidinkinase (TK) erhöht ist; gegebenenfalls wobei die Prognose der Person, wenn diese mit einem oder mehreren Immun-Checkpoint-Inhibitoren weiterbehandelt wird, positiv ist (zum Beispiel wird die Person ein progressionsfreies Überleben von mehr als 24 Monaten haben) und/oder der Patient zur Weiterbehandlung mit einem oder mehreren Immun-Checkpoint-Inhibitoren stratifiziert wird, wenn die in Schritt (b) gemessene Aktivität und/oder Konzentration der Thymidinkinase (TK) niedriger als ein Cut-off-Wert ist, und wobei die in Schritt (d) gemessene Aktivität und/oder Konzentration der Thymidinkinase (TK) im Vergleich zu der in Schritt (b) gemessenen Aktivität und/oder Konzentration der Thymidinkinase (TK) um mindestens 100 % erhöht ist.

8. Verfahren nach irgendeinem der vorhergehenden Ansprüche, wobei die Prognose der Person, wenn diese mit einem oder mehreren Immun-Checkpoint-Inhibitoren weiterbehandelt wird, negativ ist (zum Beispiel würde die Person ein progressionsfreies Überleben von weniger als 6 Monaten haben) und/oder der Patient für keine Weiterbehandlung oder Behandlung mit einer alternativen Krebstherapie zu einem oder mehreren Immun-Checkpoint-Inhibitoren stratifiziert wird, wenn die in Schritt (b) gemessene Aktivität und/oder Konzentration der Thymidinkinase (TK) größer als oder gleich einem Cut-off-Wert ist, und wobei die in Schritt (d) gemessene Aktivität und/oder Konzentration der Thymidinkinase (TK) im Vergleich zu der in Schritt (b) gemessenen Aktivität und/oder Konzentration der Thymidinkinase (TK) nicht erhöht ist.

9. Verfahren nach irgendeinem der Ansprüche 6-8, wobei die Aktivität der Thymidinkinase (TKa) in Schritt (b) gemessen wird und der Cut-off-Wert 550 DuA +/- 20 % beträgt.

10. Verfahren nach irgendeinem der vorhergehenden Ansprüche, wobei das Verfahren ferner die folgenden Schritte umfasst:

(e) Bereitstellen einer oder mehrerer Kontrollproben; und
(f) Messen der Aktivität und/oder Konzentration der Thymidinkinase (TK) in der in Schritt (e) bereitgestellten Probe;
wobei die Prognose der Person, wenn diese mit einem oder mehreren Immun-Checkpoint-Inhibitoren weiterbehandelt wird, durch Vergleichen der Messung in Schritt (f) mit der Messung in Schritt (b) und/oder Schritt (d) bestimmt wird; gegebenenfalls wobei die eine oder mehrere Kontrollproben von einer Person mit Krebs erhalten wurden, bevor sie anschließend einer erfolgreichen Behandlung mit einem oder mehreren Immun-Checkpoint-Inhibitoren unterzogen wurde.

11. Verfahren nach Anspruch 10, wobei die Prognose der Person, wenn diese mit einem oder mehreren Immun-Checkpoint-Inhibitoren weiterbehandelt wird, positiv ist und/oder der Patient zur Weiterbehandlung mit einem oder mehreren Immun-Checkpoint-Inhibitoren stratifiziert wird, wenn die in Schritt (b) gemessene Aktivität und/oder Konzentration der Thymidinkinase (TK) der in Schritt (f) gemessenen Aktivität der Thymidinkinase (TKa) entspricht oder niedriger als diese ist,

gegebenenfalls wobei die eine oder mehrere Kontrollproben von einer Person mit Krebs erhalten wurden, bevor sie anschließend einer erfolglosen Behandlung mit einem oder mehreren Immun-Checkpoint-Inhibitoren unterzogen wurde; und/oder

wobei die Prognose der Person, wenn diese mit einem oder mehreren Immun-Checkpoint-Inhibitoren weiterbehandelt wird, negativ ist und/oder der Patient für keine Weiterbehandlung oder Behandlung mit einer alternativen Krebstherapie zu einem oder mehreren Immun-Checkpoint-Inhibitoren stratifiziert wird, wenn die in Schritt (b) gemessene Aktivität und/oder Konzentration der Thymidinkinase (TK) höher als die in Schritt (f) gemessene Aktivität und/oder Konzentration der Thymidinkinase (TK) ist.

12. Verfahren nach irgendeinem der vorhergehenden Ansprüche, das ferner den folgenden Schritt umfasst:

(g) Messen der Aktivität und/oder Konzentration der Laktatdehydrogenase (LDH) in der in Schritt (a) bereitgestellten Probe;

wobei die in Schritt (g) gemessene Aktivität und/oder Konzentration der LDH zusätzlich auf die Prognose der Person hindeutet, wenn diese anschließend mit einem oder mehreren Immun-Checkpoint-Inhibitoren behandelt wird; gegebenenfalls wobei die Prognose der Person, wenn diese mit einem oder mehreren Immun-Checkpoint-Inhibitoren behandelt oder weiterbehandelt wird, positiv ist (zum Beispiel wird die Person ein progressionsfreies Überleben von mehr als 24 Monaten haben) und/oder der Patient zur Behandlung oder Weiterbehandlung mit einem oder mehreren Immun-Checkpoint-Inhibitoren stratifiziert wird, wenn die in Schritt (g) gemessene Aktivität und/oder Konzentration der LDH niedriger als ein Cut-off-Wert ist; gegebenenfalls wobei die Aktivität der LDH gemessen wird und der Cut-off-Wert zwischen 2 und 6 Mikrokat/L, zum Beispiel zwischen 3 und 5 Mikrokat/L, ausgewählt ist oder zum Beispiel 4 Mikrokat/L beträgt.

13. Ein oder mehrere Immun-Checkpoint-Inhibitoren zur Verwendung bei der Behandlung von Krebs bei einem Patienten, wobei der Patient zur Weiterbehandlung mit einem oder mehreren Immun-Checkpoint-Inhibitoren unter Verwendung eines Verfahrens zum Stratifizieren eines Patienten gemäß irgendeinem der Ansprüche 2-12 ausgewählt worden ist, wobei der Patient zur Weiterbehandlung aufgrund einer Serum- oder Plasma-TK-Aktivität und/oder -Konzentration 1-4 Wochen nach der Behandlung mit einem ICI-Inhibitor ausgewählt wird, die im Vergleich zu seiner Serum- oder Plasma-TK-Aktivität und/oder -Konzentration vor der ICI-Behandlung erhöht ist; gegebenenfalls wobei die Person zur Weiterbehandlung aufgrund einer Serum- oder Plasma-TK-Aktivität und/oder -Konzentration vor der ICI-Behandlung ausgewählt wird, die niedriger als ein Cut-off-Wert ist.

14. Verfahren oder Immun-Checkpoint-Inhibitor zur Verwendung nach irgendeinem der vorhergehenden Ansprüche, wobei:

(i) es sich um die Aktivität der Thymidinkinase (TKa) handelt, die gemessen wird;
(ii) der Krebs ein Krebs ist, der zur Behandlung mit einem Immun-Checkpoint-Inhibitor zugelassen ist;
(iii) der Krebs ausgewählt ist aus der Gruppe bestehend aus: Melanom, Brustkrebs und/oder Eierstockkrebs;
(iv) der eine oder die mehreren Immun-Checkpoint-Inhibitoren ausgewählt sind aus der Liste bestehend aus: CTLA-4-Inhibitor, PD-1-Inhibitor, PD-L1-Inhibitor, LAG-3-Inhibitor;
(v) die Person oder der Patient ein Mensch ist; und/oder
(vi) die Probe eine Plasma- oder Serumprobe ist.

15. Verwendung der Thymidinkinase: (i) als prognostischer Biomarker für Personen mit Krebs, die derzeit mit Immun-Checkpoint-Inhibitoren behandelt werden; oder (ii) zum Stratifizieren von Patienten mit Krebs zur Weiterbehandlung mit Immun-Checkpoint-Inhibitoren; wobei die Prognose der Person, wenn diese mit einem oder mehreren Checkpoint-Inhibitoren weiterbehandelt wird, positiv ist und/oder wobei der Patient zur Weiterbehandlung mit einem oder mehreren Immun-Checkpoint-Inhibitoren stratifiziert wird, wenn die in Schritt (d) gemessene Aktivität und/oder Konzentration der Thymidinkinase (TK) im Vergleich zu der in Schritt (b) gemessenen Aktivität und/oder Konzentration der Thymidinkinase (TK) erhöht ist.

**Revendications**

1. Procédé permettant la détermination du pronostic d'un individu atteint d'un cancer et actuellement traité avec un ou plusieurs inhibiteurs de points de contrôle immunitaire, le procédé comprenant ou étant constitué des étapes consistant à :

a) la fourniture d'un échantillon obtenu de l'individu avant le traitement avec un ou plusieurs inhibiteurs de points de contrôle immunitaire ;
b) la mesure de l'activité et/ou de la concentration de la thymidine kinase (TK) dans l'échantillon ;
c) la fourniture d'un échantillon obtenu de l'individu 1 à 4 semaines après le premier traitement avec un ou plusieurs inhibiteurs de points de contrôle immunitaire ; et
d) la mesure de l'activité et/ou de la concentration de la thymidine kinase (TK) dans l'échantillon fourni à l'étape (c) ;

dans lequel l'activité et/ou la concentration de thymidine kinase (TK) mesurée aux étapes (b) et (d) est indicative du pronostic de l'individu en cas de traitement supplémentaire avec un ou plusieurs inhibiteurs de points de contrôle immunitaire, dans lequel le pronostic de l'individu en cas de traitement supplémentaire avec un ou plusieurs inhibiteurs de points de contrôle est positif lorsque l'activité et/ou la concentration de thymidine kinase (TK) mesurée à l'étape (d) est augmentée par rapport à l'activité et/ou à la concentration de thymidine kinase (TK) mesurée à l'étape (b).

2. Procédé selon la revendication 1, dans lequel le procédé consiste à stratifier un ou plusieurs patients atteints de cancer en vue d'un traitement ultérieur avec un ou plusieurs inhibiteurs de points de contrôle immunitaire.

3. Procédé permettant la stratification d'un patient atteint d'un cancer en vue d'un traitement ultérieur avec un ou plusieurs inhibiteurs de points de contrôle immunitaire, le procédé comprenant ou constitué des étapes consistant à :

i) la mise en œuvre du procédé selon l'une quelconque revendication précédente ; et
ii) la stratification du patient en vue d'un traitement ultérieur avec un ou plusieurs inhibiteurs de points de contrôle immunitaire ou une thérapie anticancéreuse alternative en fonction du résultat de l'étape (i), dans lequel le patient est stratifié en vue d'un traitement ultérieur avec un ou plusieurs inhibiteurs de points de contrôle immunitaire lorsque l'activité et/ou la concentration de la thymidine kinase (TK) mesurée à l'étape (d) est augmentée par rapport à l'activité et/ou à la concentration de thymidine kinase (TK) mesurée à l'étape (b).

4. Procédé selon l'une quelconque revendication précédente dans lequel le pronostic de l'individu, en cas de traitement supplémentaire avec un ou plusieurs inhibiteurs de points de contrôle, est positif (par exemple, l'individu aura une survie sans progression de plus de 24 mois) et/ou le patient est stratifié pour un traitement ultérieur avec un ou plusieurs inhibiteurs de points de contrôle immunitaire lorsque l'activité et/ou la concentration de thymidine kinase (TK) mesurée à l'étape (d) est augmentée d'au moins 100 % par rapport à l'activité et/ou à la concentration de thymidine kinase (TK) mesurée à l'étape (b).

5. Procédé selon l'une quelconque revendication précédente dans lequel le pronostic de l'individu, en cas de traitement supplémentaire avec un ou plusieurs inhibiteurs de points de contrôle immunitaire, est positif et/ou le patient est stratifié pour un traitement supplémentaire avec un ou plusieurs inhibiteurs de points de contrôle immunitaire lorsque l'activité et/ou la concentration de thymidine kinase (TK) mesurée à l'étape (b) est inférieure à une valeur seuil.

6. Procédé selon l'une quelconque revendication précédente dans lequel le pronostic de l'individu, en cas de traitement supplémentaire avec un ou plusieurs inhibiteurs de points de contrôle immunitaire, est négatif (par exemple, l'individu aurait une survie sans progression de moins de 6 mois) et/ou le patient est stratifié pour ne plus recevoir de traitement ou un traitement anticancéreux alternatif à un ou plusieurs inhibiteurs de points de contrôle immunitaire lorsque l'activité et/ou la concentration de la thymidine kinase (TK) mesurée à l'étape (b) est supérieure ou égale à une valeur seuil.

7. Procédé selon l'une quelconque revendication précédente dans lequel le pronostic de l'individu en cas de traitement supplémentaire avec un ou plusieurs inhibiteurs de points de contrôle immunitaire est positif (par exemple, l'individu aura une survie sans progression d'au moins 6 mois) et/ou le patient est stratifié pour un traitement supplémentaire avec un ou plusieurs inhibiteurs de points de contrôle immunitaire lorsque l'activité et/ou la concentration de thymidine kinase (TK) mesurée à l'étape (b) est inférieure à une valeur seuil, et dans lequel l'activité et/ou la concentration de thymidine kinase (TK) mesurée à l'étape (d) est augmentée par rapport à l'activité et/ou la concentration de thymidine kinase (TK) mesurée à l'étape (b) ; éventuellement, dans lequel le pronostic de l'individu, en cas de traitement supplémentaire avec un ou plusieurs inhibiteurs de points de contrôle immunitaire, est positif (par exemple, l'individu aura une survie sans progression de plus de 24 mois) et/ou le patient est stratifié pour un traitement supplémentaire avec un ou plusieurs inhibiteurs de points de contrôle immunitaire lorsque l'activité et/ou la concentration de thymidine kinase (TK) mesurée à l'étape (b) est inférieure à une valeur seuil, et dans lequel l'activité et/ou la concentration de thymidine kinase (TK) mesurée à l'étape (d) est augmentée d'au moins 100 % par rapport à l'activité et/ou la concentration de thymidine kinase (TK) mesurée à l'étape (b).

8. Procédé selon l'une quelconque revendication précédente dans lequel le pronostic de l'individu en cas de traitement supplémentaire avec un ou plusieurs inhibiteurs de points de contrôle immunitaire est négatif (par exemple, l'individu aurait une survie sans progression de moins de 6 mois) et/ou le patient est stratifié pour ne plus recevoir de traitement ou un traitement anticancéreux alternatif à un ou plusieurs inhibiteurs de points de contrôle immunitaire lorsque l'activité et/ou la concentration de thymidine kinase (TK) mesurée à l'étape (b) est supérieure ou égale à une valeur seuil, et dans lequel l'activité et/ou la concentration de thymidine kinase (TK) mesurée à l'étape (d) n'est pas augmentée par rapport à l'activité et/ou à la concentration de thymidine kinase (TK) mesurée à l'étape (b).

9. Procédé selon l'une quelconque des revendications 6 à 8, dans lequel l'activité de la thymidine kinase (TKa) est mesurée à l'étape (b) et la valeur seuil est de 550 DuA +/- 20 %.

10. Procédé selon l'une quelconque revendication précédente dans lequel le procédé comprend en outre les étapes consistant à :

(e) la fourniture d'un ou plusieurs échantillons de contrôle ; et
(f) la mesure de l'activité et/ou de la concentration de la thymidine kinase (TK) dans l'échantillon fourni à l'étape (e) ;
dans lequel le pronostic de l'individu en cas de traitement supplémentaire avec un ou plusieurs inhibiteurs de points de contrôle immunitaire est déterminé en comparant la mesure à l'étape (f) avec la mesure à l'étape (b) et/ou à l'étape (d) ; éventuellement dans lequel le ou les échantillons de contrôle ont été obtenus à partir d'un individu atteint d'un cancer avant de subir un traitement réussi avec un ou plusieurs inhibiteurs de points de contrôle immunitaire.

11. Procédé selon la revendication 10 dans lequel le pronostic de l'individu en cas de traitement supplémentaire avec un ou plusieurs inhibiteurs de points de contrôle immunitaire est positif et/ou le patient est stratifié pour un traitement supplémentaire avec un ou plusieurs inhibiteurs de points de contrôle immunitaire lorsque l'activité et/ou la concentration de thymidine kinase (TK) mesurée à l'étape (b) correspond à ou est inférieure à l'activité de thymidine kinase (TKa) mesurée à l'étape (f), éventuellement

dans lequel le ou les échantillons de contrôle ont été obtenus à partir d'un individu atteint d'un cancer avant de subir un traitement infructueux avec un ou plusieurs inhibiteurs de points de contrôle immunitaire ; et/ou
dans lequel le pronostic de l'individu en cas de traitement supplémentaire avec un ou plusieurs inhibiteurs de points de contrôle immunitaire est négatif et/ou le patient est stratifié pour ne plus recevoir de traitement ou un traitement anticancéreux alternatif à un ou plusieurs inhibiteurs de points de contrôle immunitaires lorsque l'activité et/ou la concentration de thymidine kinase (TK) mesurée à l'étape (b) est supérieure à l'activité et/ou à la concentration de thymidine kinase (TK) mesurée à l'étape (f).

12. Procédé selon l'une quelconque revendication précédente dans lequel le procédé comprend en outre l'étape consistant à :

g) la mesure de l'activité et/ou la concentration de lactate déshydrogénase (LDH) dans l'échantillon fourni à l'étape (a) ;
dans lequel l'activité et/ou la concentration de LDH mesurée à l'étape (g) sont en outre indicatives du pronostic de l'individu s'il est ensuite traité avec un ou plusieurs inhibiteurs de points de contrôle immunitaire ; éventuellement, dans lequel le pronostic de l'individu s'il est traité ou en outre traité avec un ou plusieurs inhibiteurs de points de contrôle immunitaire est positif (par exemple, l'individu aura une survie sans progression de plus de 24 mois) et/ou le patient est stratifié pour un traitement ou un traitement ultérieur avec un ou plusieurs inhibiteurs de points de contrôle immunitaire lorsque l'activité et/ou la concentration de LDH mesurée à l'étape (g) est inférieure à une valeur seuil ; éventuellement, dans lequel l'activité de la LDH est mesurée et la valeur seuil est choisie entre 2 et 6 mikrokat/L, par exemple entre 3 et 5 mikrokat/L, ou par exemple est de 4 mikrokat/L.

13. Un ou plusieurs inhibiteurs de points de contrôle immunitaire pour usage dans le traitement du cancer chez un patient, dans lequel le patient a été sélectionné pour un traitement supplémentaire avec un ou plusieurs inhibiteurs de points de contrôle immunitaire à l'aide d'un procédé de stratification d'un patient selon l'une quelconque des revendications 2 à 12,

dans lequel le patient est sélectionné pour un traitement supplémentaire parce qu'il présente une activité et/ou une concentration de TK dans le sérum ou le plasma 1 à 4 semaines après le traitement par un inhibiteur d'ICI qui est augmenté par rapport à son activité et/ou sa concentration de TK dans le sérum ou le plasma avant le traitement par ICI ; éventuellement, dans lequel l'individu est sélectionné pour un traitement supplémentaire parce qu'il présente également une activité et/ou une concentration de TK dans le sérum ou le plasma avant le traitement par ICI qui est inférieur à une valeur seuil.

14. Procédé ou inhibiteur de point de contrôle immunitaire pour usage selon l'une quelconque revendication précédente dans lequel :

(i) c'est l'activité de la thymidine kinase (TKa) qui est mesurée ;
(ii) le cancer est un cancer approuvé pour un traitement avec un inhibiteur de point de contrôle immunitaire ;
(iii) le cancer est choisi dans le groupe comprenant : le mélanome, le cancer du sein et/ou le cancer de l'ovaire ;
(iv) le ou les inhibiteurs de points de contrôle immunitaire sont choisis dans la liste constituée de : l'inhibiteur de CTLA-4, l'inhibiteur de PD-1, l'inhibiteur de PD-L1, l'inhibiteur de LAG-3 ;
(v) l'individu ou le patient est un être humain ; et/ou
(vi) l'échantillon est un échantillon de plasma ou de sérum.

15. Utilisation de la thymidine kinase : (i) comme biomarqueur pronostique pour des individus atteints de cancer qui sont actuellement traités avec des inhibiteurs de points de contrôle immunitaire ; ou (ii) pour stratifier des patients atteints de cancer en vue d'un traitement supplémentaire avec des inhibiteurs de points de contrôle immunitaire ; dans lequel le pronostic de l'individu est positif s'il est traité ultérieurement avec un ou plusieurs inhibiteurs de points de contrôle et/ou dans lequel le patient est stratifié en vue d'un traitement supplémentaire avec un ou plusieurs inhibiteurs de points de contrôle immunitaire lorsque l'activité et/ou la concentration de thymidine kinase (TK) mesurée à l'étape (d) est augmentée par rapport à l'activité et/ou à la concentration de thymidine kinase (TK) mesurée à l'étape (b).

Figure 1

Figure 2

Figure 2 (cont'd)

Figure 2 (cont'd)

Figure 3

# PFS

| Term | HR | 95%-CI | HR | P-value | Concordance |
|---|---|---|---|---|---|
| TKa (high vs. low) | 1.66 | (0.97 - 2.83) | | 0.066 | |
| Age (old vs. young) | 1.53 | (0.88 - 2.68) | | 0.134 | 59.96 |
| TKa (high vs. low) | 1.91 | (1.12 - 3.26) | | 0.017 | |
| Sex (female vs. male) | 0.79 | (0.45 - 1.37) | | 0.397 | 58.93 |
| TKa (high vs. low) | 1.24 | (0.66 - 2.34) | | 0.505 | |
| ECOG (≥1 vs. 0) | 2.29 | (1.13 - 4.65) | | 0.022 | 61.11 |
| TKa (high vs. low) | 1.57 | (0.90 - 2.71) | | 0.109 | |
| Tumor stage (M1a-b vs. M1c-d) | 1.65 | (0.96 - 2.84) | | 0.071 | 62.46 |
| TKa (high vs. low) | 1.32 | (0.73 - 2.37) | | 0.354 | |
| LDH (elevated vs. normal) | 1.83 | (1.03 - 3.27) | | 0.040 | 61.04 |

0.4  0.6  0.8  1.0  1.4  2.0  3.0  4.0  6.0

## Figure 4

EP 4 500 177 B1

# OS

| Term | HR | 95%-CI | HR | P-value | Concordance |
|---|---|---|---|---|---|
| TKa (high vs. low) | 1.89 | (1.04 - 3.46) | | 0.038 | |
| Age (old vs. young) | 2.18 | (1.12 - 4.25) | | 0.023 | 64.59 |
| TKa (high vs. low) | 2.23 | (1.23 - 4.07) | | 0.009 | |
| Sex (female vs. male) | 1.04 | (0.57 - 1.92) | | 0.891 | 58.18 |
| TKa (high vs. low) | 1.41 | (0.69 - 2.89) | | 0.346 | |
| ECOG (≥1 vs. 0) | 2.76 | (1.25 - 6.06) | | 0.012 | 65.05 |
| TKa (high vs. low) | 1.90 | (1.04 - 3.49) | | 0.038 | |
| Tumor stage (M1a-b vs. M1c-d) | 1.96 | (1.06 - 3.63) | | 0.031 | 64.52 |
| TKa (high vs. low) | 1.49 | (0.77 - 2.87) | | 0.234 | |
| LDH (elevated vs. normal) | 2.63 | (1.32 - 5.24) | | 0.006 | 66.08 |

0.4  0.6  0.8  1.0  1.4  2.0  3.0  4.0  6.0

## Figure 4 (contd)

EP 4 500 177 B1

Figure 5

Figure 6

Figure 7

EP 4 500 177 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011142719 A

**Non-patent literature cited in the description**

- **COSTA SVEDMAN et al.** *Cancers*, 2022, vol. 14 (3), 702
- **MCCARTNEY et al.** *Clinical Cancer Research*, 2020, vol. 26 (9), 2131
- **KIM et al.** *Clin Cancer Res*, 2020, vol. 25 (7)
- **SCHWARTZ et al.** *J. Nucl. Med.*, 2003, vol. 44, 2027-2032
- **NISMAN et al.** *Clinical Chemistry and Laboratory Medicine*, 2013, vol. 51 (2), 439-47
- **BAGEGNI et al.** *Breast Cancer Res.*, 2017, vol. 19 (1), 123
- **STÅLHANDSKE et al.** *Analytical Biochemistry*, 2013, vol. 432, 155-164
- **TWOMEY** ; **ZHANG**. *The AAPS Journal*, 2021, vol. 23, 39
- **HODI FS** ; **O'DAY SJ** ; **MCDERMOTT DF** ; **WEBER RW** ; **SOSMAN JA** ; **HAANEN JB et al.** Improved survival with ipilimumab in patients with metastatic melanoma. *N Engl J Med.*, 2010, vol. 363, 711-23
- **ROBERT C** ; **LONG GV** ; **BRADY B** ; **DUTRIAUX C** ; **MAIO M** ; **MORTIER L et al.** Nivolumab in previously untreated melanoma without BRAF mutation. *N Engl J Med.*, 2015, vol. 372, 320-30
- **ROBERT C** ; **SCHACHTER J** ; **LONG GV** ; **ARANCE A** ; **GROB JJ** ; **MORTIER L et al.** Pembrolizumab versus Ipilimumab in Advanced Melanoma. *N Engl J Med.*, 2015, vol. 372, 2521-32
- **LARKIN J** ; **CHIARION-SILENI V** ; **GONZALEZ R** ; **GROB JJ** ; **COWEY CL** ; **LAO CD et al.** Combined Nivolumab and Ipilimumab or Monotherapy in Untreated Melanoma. *N Engl J Med.*, 2015, vol. 373, 23-34
- **ROBERT C** ; **KARASZEWSKA B** ; **SCHACHTER J** ; **RUTKOWSKI P** ; **MACKIEWICZ A** ; **STROIAKOVS-KI D et al.** Improved overall survival in melanoma with combined dabrafenib and trametinib. *N Engl J Med.*, 2015, vol. 372, 30-9

- **DUMMER R** ; **ASCIERTO PA** ; **GOGAS HJ** ; **ARANCE A** ; **MANDALA M** ; **LISZKAY G et al.** Encorafenib plus binimetinib versus vemurafenib or encorafenib in patients with BRAF-mutant melanoma (COLUMBUS): a multicentre, open-label, randomised phase 3 trial.. *Lancet Oncol.*, 2018, vol. 19, 603-15
- **SCHWARTZ JL** ; **TAMURA Y** ; **JORDAN R** ; **GRIERSON JR** ; **KROHN KA.** Monitoring tumor cell proliferation by targeting DNA synthetic processes with thymidine and thymidine analogs. *J Nucl Med.*, 2003, vol. 44, 2027-32
- **BITTER EE** ; **TOWNSEND MH** ; **ERICKSON R** ; **ALLEN C** ; **O'NEILL KL.** Thymidine kinase 1 through the ages: a comprehensive review. *Cell Biosci.*, 2020, vol. 10, 138
- **BAGEGNI N** ; **THOMAS S** ; **LIU N** ; **LUO J** ; **HOOG J** ; **NORTHFELT DW et al.** Serum thymidine kinase 1 activity as a pharmacodynamic marker of cyclin-dependent kinase 4/6 inhibition in patients with early-stage breast cancer receiving neoadjuvant palbociclib. *Breast Cancer Res.*, 2017, vol. 19, 123
- **MCCARTNEY A** ; **BONECHI M** ; **DE LUCA F** ; **BIAGIONI C** ; **CURIGLIANO G** ; **MORETTI E et al.** Plasma Thymidine Kinase Activity as a Biomarker in Patients with Luminal Metastatic Breast Cancer Treated with Palbociclib within the TREnd Trial.. *Clin Cancer Res.*, 2020, vol. 26, 2131-9
- **CABEL L** ; **ROSENBLUM D** ; **LEREBOURS F** ; **BRAIN E** ; **LOIRAT D** ; **BERGQVIST M et al.** Plasma thymidine kinase 1 activity and outcome of ER+ HER2- metastatic breast cancer patients treated with palbociclib and endocrine therapy. *Breast Cancer Res.*, 2020, vol. 22, 98
- **FELIX K** ; **HINZ U** ; **DOBIASCH S** ; **HACKERT T** ; **BERGMANN F** ; **NEUMULLER M et al.** Preoperative Serum Thymidine Kinase Activity as Novel Monitoring, Prognostic, and Predictive Biomarker in Pancreatic Cancer. *Pancreas.*, 2018, vol. 47, 72-9

- **MCCARTNEY A** ; **BIAGIONI C** ; **SCHIAVON G** ; **BERGQVIST M** ; **MATTSSON K** ; **MIGLIACCIO I et al.** Prognostic role of serum thymidine kinase 1 activity in patients with hormone receptor-positive metastatic breast cancer: Analysis of the randomised phase III Evaluation of Faslodex versus Exemestane Clinical Trial (EFECT). *Eur J Cancer.*, 2019, vol. 114, 55-66
- **NISMAN B** ; **APPELBAUM L** ; **YUTKIN V** ; **NECHUSHTAN H** ; **HUBERT A** ; **UZIELY B et al.** Serum Thymidine Kinase 1 Activity Following Nephrectomy for Renal Cell Carcinoma and Radiofrequency Ablation of Metastases to Lung and Liver.. *Anticancer Res.*, 2016, vol. 36, 1791-7
- **NISMAN B** ; **NECHUSHTAN H** ; **BIRAN H** ; **GANTZ-SOROTSKY H** ; **PELED N** ; **GRONOWITZ S et al.** Serum thymidine kinase 1 activity in the prognosis and monitoring of chemotherapy in lung cancer patients: a brief report.. *J Thorac Oncol.*, 2014, vol. 9, 1568-72
- **GERSHENWALD JE** ; **SCOLYER RA** ; **HESS KR** ; **SONDAK VK** ; **LONG GV** ; **ROSS MI et al.** Melanoma staging: Evidence-based changes in the American Joint Committee on Cancer eighth edition cancer staging manual.. *CA Cancer J Clin.*, 2017, vol. 67, 472-92
- **EISENHAUER EA** ; **THERASSE P** ; **BOGAERTS J** ; **SCHWARTZ LH** ; **SARGENT D** ; **FORD R et al.** New response evaluation criteria in solid tumours: revised RECIST guideline (version 1.1).. *Eur J Cancer.*, 2009, vol. 45, 228-47
- **BJOHLE J** ; **BERGQVIST J** ; **GRONOWITZ JS** ; **JOHANSSON H** ; **CARLSSON L** ; **EINBEIGI Z et al.** Serum thymidine kinase activity compared with CA 15-3 in locally advanced and metastatic breast cancer within a randomized trial.. *Breast Cancer Res Treat.*, 2013, vol. 139, 751-8
- **PALMER SR** ; **ERICKSON LA** ; **ICHETOVKIN I** ; **KNAUER DJ** ; **MARKOVIC SN.** Circulating serologic and molecular biomarkers in malignant melanoma. *Mayo Clin Proc.*, 2011, vol. 86, 981-90
- **FUJII T** ; **NAING A** ; **ROLFO C** ; **HAJJAR J.** Biomarkers of response to immune checkpoint blockade in cancer treatment. *Crit Rev Oncol Hematol.*, 2018, vol. 130, 108-20
- **PICO DE COANA Y** ; **WOLODARSKI M** ; **VAN DER HAAR AVILA I** ; **NAKAJIMA T** ; **RENTOULI S** ; **LUNDQVIST A et al.** PD-1 checkpoint blockade in advanced melanoma patients: NK cells, monocytic subsets and host PD-L1 expression as predictive biomarker candidates. *Oncoimmunology*, 2020, vol. 9, 1786888
- **HUGO W** ; **ZARETSKY JM** ; **SUN L** ; **SONG C** ; **MORENO BH** ; **HU-LIESKOVAN S et al.** Genomic and Transcriptomic Features of Response to Anti-PD-1 Therapy in Metastatic Melanoma. *Cell.*, 2016, vol. 165, 35-44
- **SNYDER A** ; **MAKAROV V** ; **MERGHOUB T** ; **YUAN J** ; **ZARETSKY JM** ; **DESRICHARD A et al.** Genetic basis for clinical response to CTLA-4 blockade in melanoma. *N Engl J Med.*, 2014, vol. 371, 2189-99
- **ROZEMAN EA** ; **HOEFSMIT EP** ; **REIJERS ILM** ; **SAW RPM** ; **VERSLUIS JM** ; **KRIJGSMAN O et al.** Survival and biomarker analyses from the OpACIN-neo and OpACIN neoadjuvant immunotherapy trials in stage III melanoma. *Nat Med.*, 2021, vol. 27, 256-63
- **GRASSO CS** ; **TSOI J** ; **ONYSHCHENKO M** ; **ABRIL-RODRIGUEZ G** ; **ROSS-MACDONALD P** ; **WIND-ROTOLO M et al.** Conserved Interferon-gamma Signaling Drives Clinical Response to Immune Checkpoint Blockade Therapy in Melanoma. *Cancer Cell.*, 2020, vol. 38, 500-15 e3
- **KAMINSKA P** ; **BUSZKA K** ; **ZABEL M** ; **NOWICKI M** ; **ALIX-PANABIERES C** ; **BUDNA-TUKAN J.** Liquid Biopsy in Melanoma: Significance in Diagnostics, Prediction and Treatment Monitoring. *Int J Mol Sci.*, 2021, vol. 22
- **GOPALAKRISHNAN V** ; **SPENCER CN** ; **NEZI L** ; **REUBEN A** ; **ANDREWS MC** ; **KARPINETS TV et al.** Gut microbiome modulates response to anti-PD-1 immunotherapy in melanoma patients. *Science.*, 2018, vol. 359, 97-103
- **KIM KH** ; **CHO J** ; **KU BM** ; **KOH J** ; **SUN JM** ; **LEE SH** ; **AHN JS** ; **CHEON J** ; **MIN YJ** ; **PARK SH.** The First-week Proliferative Response of Peripheral Blood PD-1+CD8+ T Cells Predicts the Response to Anti-PD-1 Therapy in Solid Tumors. *Clin Cancer Res.*, 01 April 2019, vol. 25 (7), 2144-2154